(19) Europäisches Patentamt
European Patent Office
Office européen des brevets

(11) **EP 1 828 375 B1**

(12) **EUROPEAN PATENT SPECIFICATION**

(45) Date of publication and mention of the grant of the patent:
**07.05.2008 Bulletin 2008/19**

(21) Application number: **04821402.7**

(22) Date of filing: **21.12.2004**

(51) Int Cl.:
*C12N 1/21* (2006.01)      *C07K 14/475* (2006.01)

(86) International application number:
**PCT/IN2004/000393**

(87) International publication number:
**WO 2006/067793 (29.06.2006 Gazette 2006/26)**

(54) **LOW CELL DENSITY FERMENTATION PROCESS FOR THE PRODUCTION OF HETEROLOGOUS RECOMBINANT PROTEINS IN MICROORGANISMS**

FERMENTATIONSVERFAHREN MIT GERINGER ZELLDICHTE ZUR PRODUKTION HETEROLOGER REKOMBINANTER PROTEINE IN MIKROORGANISMEN

PROCEDE DE FERMENTATION A FAIBLE DENSITE CELLULAIRE POUR LA PRODUCTION DE PROTEINES RECOMBINANTES HETEROLOGUES DANS DES MICROORGANISMES

(84) Designated Contracting States:
**AT BE BG CH CY CZ DE DK EE ES FI FR GB GR HU IE IS IT LI LT LU MC NL PL PT RO SE SI SK TR**

(43) Date of publication of application:
**05.09.2007 Bulletin 2007/36**

(73) Proprietor: **USV Limited**
**Mumbai 400088,**
**Maharashtra (IN)**

(72) Inventors:
• **NIPHADKAR, Milind Prabhakar**
**USV Limited B.S.D.**
**Mumbai 400 088, Maharashtra (IN)**
• **NAZARETH, Genevieve Suzie**
**USV Limited B.S.D.**
**Mumbai 400 088, Maharashtra (IN)**
• **SURLIKAR, Neelesh Ramesh**
**USV Limited B.S.D.**
**Mumbai 400 088, Maharashtra (IN)**
• **BORBHUIYA, Monsur Ahmed**
**USV Limited B.S.D.**
**Mumbai 400 088, Maharashtra (IN)**

• **SANKARARAMAN, Uma**
**USV Limited B.S.D.**
**Mumbai 400 088, Maharashtra (IN)**
• **MAITI, Dipanwita**
**USV Limited B.S.D.**
**Mumbai 400 088, Maharashtra (IN)**
• **RAO, Laxmi, Srinivas**
**USV Limited B.S.D.**
**Mumbai 400 088, Maharashtra (IN)**
• **PAUL, Saptarshi**
**USV Limited B.S.D.**
**Mumbai 400 088, Maharashtra (IN)**
• **MISHRA, Shrikant**
**USV Limited B.S.D.**
**Mumbai 400 088, Maharashtra (IN)**

(74) Representative: **Eddowes, Simon**
**W. P. Thompson & Co.**
**Eastcheap House**
**Central Approach**
**Letchworth**
**Hertfordshire SG6 3DS (GB)**

(56) References cited:
**WO-A1-01/42420        US-A- 5 595 905**
**US-A- 5 714 348**

**Description**

**Field of Invention**

This invention relates to a low cell density fermentation process for the production of heterologous recombinant proteins in microorganisms.

**Prior Art**

Fermentation process comprises growing or cultivating microbial cell cultures under controlled conditions for the production of metabolic by-products such as antibiotics, wine or beer. In recent times, fermentation is also used to produce a number of chemical substances such as proteins possessing pharmacological activity. Fermentation process is carried out in two steps namely growth phase and production phase. The growth phase comprises cultivating host cells transformed with a vector carrying genetic material coding for the desired proteins and an inducible or constitutive promoter in a nutrient medium comprising a carbon source such as glucose, maltose, sorbitol or glycerol; a nitrogen source such as ammonia, nitrate, yeast extract, casamino acids or peptone; and inorganic phosphates and trace elements such as salts of iron, copper, calcium, cobalt, zinc, manganese or molybdenum and optionally antibiotics. During the production phase, the promoter is induced with an inducer for the expression or production of the proteins. During the production phase the growth and metabolic rate of the cell cultures reduce due to diversion of both carbon and energy for the fromation of induced protein leading to a change in central carbon metabolism (Schweder T et al, "Role of the general stress response during strong overexpression of a heterologous gene in Escherichia coli" Appl. Microbiol Biotech 58: 330-337 (2002). This in turn leads to reduced cell viability, further reduction in growth of cell cultures and expression or yield of the desired proteins and finally termination of the fermentation. Also during fermentation, the nutrient concentration usually that of carbon source being very high, there is excessive acetic acid formation. Production of undesirable by-products such as acetate in the nutrient medium inhibits growth of the microbial cell culture ie do not allow the cells to grow to the required cell concentration. This also inhibits production or expression of proteins. Acetate formation in the cell culture may be reduced by the fed-batch technique wherein microorganisms are grown in a substrate limited medium ie controlled addition of *the* medium component which leads to products which inhibit the growth of the cells.

Fermentation processes comprising cultivation of cell cultures to achieve cell concentration of about $OD_{600}=100$ (Dry cell weight, DCW~50g/L) are generally known as low cell density fermentations. In low cell density fermentations several fed batch techniques are reported. In one such low cell density fermentations process, acetate formation is controlled by varying the concentration of carbon source made available to the cell culture during the fed batch phase ie carbon source and oxygen is made available to the cell culture in low and high quantities during the fed batch phase. PCT Publication No WO 01/42420 describes a fed-batch phase wherein cultivation of the cells is carried out by addition of carbon source by oscillation feed and/or by variation of stirring speed of the stirrer in the fermenter eg in square or sine waves. In this process growth rate of the cell culture may not be constant due to variations in the amount of carbon source made available to the cell cultures. Therefore, expression and production of the proteins may be variable and is reduced. Besides, this process requires use of expensive equipments for calculation of the requisite amount of carbon source to be made available to the cell culture during the fed batch phase. [Lin HY and Neubauer P; J Biotechnology, 79(1) : 27 - 37, 2000]. Additionally, concentration, of carbon source to be made available to the cell culture varies from protein to protein so also the host cells used in the fermentation process. Therefore, the process is very complex and complicated.

Another low cell density fermentation process is described in US Patent No 5714348 wherein cultures of oxygen dependent cells are cultivated and carbon source is made available to the cultures at the rate proportional to the oxygen uptake rate of the cells during the growth phase. When an empirical threshold value is reached during the production phase a constant concentration of carbon source is made available to the culture. During production phase oxygen uptake rate of the cells may not be, however, constant and as the carbon source concentration made available to the cells is constant metabolic rate of the cells reduces leading to reduced growth of cells and expression or yield of the desired proteins.

Predictive and feed back algorithms are also used to obtain maximum yield of proteins in low cell density fermentation process. These algorithms maintain concentration of the carbon source in nutrient medium constant during fermentation thereby controlling production of undesirable by-products such as acetic acid and allowing growth of the cells at a constant rate. (Kleman G L et al, Appl. Environ. Microbiol 57 (4) : 910 - 917, 1991). In order to define these algorithms extensive archived data for carbon source consumption rates are required. Moreover, these algorithms vary for different organisms. The process is, therefore, complex and expensive. (Kleman G L et al, Appl. Environ. Microbiol 57 (4) : 910 - 917, 1991).

US Patent No 5,595,905 relates to a fermentation process wherein the carbon source concentration in the nutrient medium is regulated by means of computer programs. This process involves withdrawal of samples of the fermentation broth at regular intervals of time, measurement of the carbon source consumption in each of the samples and prediction of the quantity of nutrient medium to be added to the fermentation broth for regulation of the carbon source concentration in the medium after comparing the rate of consumption of the carbon source of the different samples of the fermentation broth. This process is very complex and complicated. Besides, the rate of addition of the nutrient medium to the fermentation broth may vary from organism to organism. Also, the process makes use of computers with sophisticated computational capability and other equipments which are very expensive thereby rendering the process uneconomical.

PCT Publication No WO 02/40697 describes a low cell density fermentation process wherein yield of usable proteins is increased by reducing the metabolic rate of the cell culture during the production phase of the fed batch phase by decreasing the concentration of oxygen available to the cell culture. The reduction in the metabolic rate of the cell culture does not allow the cells to grow to the required concentration thereby resulting in reduced yield of usable proteins. Due to variation of metabolic rates,stresses on the cells may increase and limit the duration for which the cultivation can be effectively operated.

Another low cell density fermentation process comprises separating the growth and production phase of the fed batch phase by carrying them out in two separate fermenters using different nutrient media. The process may also involve testing of the genetic stability of the cells in the two media. The process is complex, cumbersome and expensive. There are also chances of the cell cultures being contaminated due to transfer of the cell cultures from fermenter to fermenter. [Chang C C et al; Appl. Microbiol Biotechnol; 49(5) : 531 - 537, 1998)].

In order to obtain high yield of usable proteins during low cell density fermentation, feasible quadratic programming algorithms are also employed. These algorithms may vary for different organisms and require extensive knowledge and data on the behaviour of the organism and computational techniques thereby rendering the process very complex and expensive [Chen Q et al; Appl. Biochem. Biotechnol; 51 - 52 : 449 - 461; 1995].

PCT Publication No WO 96/39523 describes a low cell density fermentation process for the production of hydrophobic proteins such as interferon - beta, wherein the nutrient medium in which the cells are cultured comprises low potassium and sodium cations and glycerol as the carbon source. As these components are used by the cells for their growth, the concentration of these ions in the nutrient medium reduces and needs to be replenished periodically. This involves measurement of the concentration of the -cations in the fermentation broth by taking out samples of the fermentation broth at regular intervals of time. The fermenter used for this fermentation process may require modification for the addition of the cations in the fermentation broth at different points of time. Due to frequent sampling of the fermentation broth there may be chances of the fermentation broth getting contaminated.

Low cell density fermentation techniques in general employ short fermentation cycles because of which fermentation time and running cost of fermentation are reduced. Consequently number of fermentations in unit time and percentage of productive fermentation are increased. Low density fermentation generates reduced biomass correspondingly reducing disposal problem thereof. At low cell fermentation densities, feed volume reduces and it is possible to achieve the desired density within volume limits imposed by the geometry of the fermenters. Low cell density fermentation techniques, however, differ depending on the host cell cultures and proteins of interest. Therefore the same low cell density fermentation process is not generally applicable to different cell cultures to produce different proteins. Also due to factors described above and due to cell concentrations achieved being low, yield and expression of proteins obtained by low cell density fermentations may be low. Generally yields of proteins are in the range of few (hundred) mgs to 1 gm (87 mg/L, Lee, J.Y., Yoon, C.S., et al, "Scale-up process for expression and renaturation of recombinant human epidermal growth factor from Escherichia coli inclusion bodies", Biotechnol Appl Biochem, 31:245-248, (2000); 150 mg/L, WO 02/077205 A2; 60 mg/L, Shimizu, N., Fukuzono, S., et al, Biotechnol. Bioeng., 38: 37-42, (1991); 600 mg/L, Tsai,L.B., Mann, M., et al, J. Ind. Microbiol. 2: 181-187, (1987).

High cell density fermentation processes are used to obtain increased yields of usable proteins by increasing the amount of fermenting cells. Fermentation processes comprising cultivation of cell cultures to achieve cell concentrations of about $OD_{600}$ 150 (DCW~80g/L) and above are generally known as high cell density fermentations. Acetic acid formation is also encountered in high cell density fermentation because of which yield and expression of proteins are hampered. Generally yields of heterologous proteins are reported in the range of 56 mgs to 5.6 gms/L (Lee S.Y., Trends. Biotech. 14: 98-105 1996). Besides these processes require specialized equipments for supply of pure oxygen and explosion protection which are very expensive. High cell density fermentation processes also make use of expensive components such as polyphosphates in the nutrient medium to avoid precipitation of inorganic phosphates (EP Patent No 0755438). These processes also generate huge amounts of biomass whose disposal is cumbersome and expensive.

OBJECTS OF THE INVENTION

An object of the invention is to provide a low cell density fermentation process which is versatile and produces heterologous recombinant proteins in microorganisms.

Another object of the invention is to provide a low cell density fermentation process which results in good yield or accumulation or production or expression of heterologous recombinant proteins.

Another object of the invention is to provide a low cell density fermentation process which uses the same feed medium for the entire fermentation process.

Another object of the invention is to provide a low cell density fermentation process which uses the same feed medium for the entire fermentation process employing a non-computational feed strategy which is simple and easy to carry out.

Another object of the invention is to provide a low cell density fermentation process which minimises formation of undesirable by-products such as acetic acid thereby allowing growth of cells and increasing production of heterologous recombinant proteins.

Another object of the invention is to provide a low cell density fermentation process which eliminates use of special equipments to control formation of undesirable by-products such as acetic acid thereby rendering the process economical and simple to carry out.

Another object of the invention is to provide a low cell density fermentation process which does not employ sophisticated equipments for supply of pure oxygen and explosion protection and is therefore further economical and simple to carry out.

Another object of the invention is to provide a low cell density fermentation process wherein cell growth and expression or production of proteins are independent of each other.

Another object of the invention is to provide a low cell density fermentation process which results in reduced biomass.

Another object of the invention is to provide a low cell density fermentation process wherein cells grow at a constant rate for the entire duration of the fermentation process.

Another object of the invention is to provide a low cell density fermentation process wherein cell growth and expression of proteins are achieved simultaneously independently during the entire fermentation process.

DETAILED DESCRIPTION OF THE INVENTION

According to the invention there is provided a low cell density fermentation process for production of heterologous proteins in microorganisms comprising

a) feeding the cell culture obtained by cultivating host microorganisms transformed with a vector carrying genetic material for the said proteins and an inducible promoter under batch fermentation conditions, with a feed medium after an $OD_{600}$ of 0.16 to 8 has been achieved or after 0 to 4 hrs from the start of the fermentation process, the feed medium comprising 5 to 30% of carbon source and 1 to 30% of nitrogen source and 0 mg to 400 mg antibiotics and 2.5 to 4.25% inorganic phosphates and trace elements, the concentration of the carbon source in the feed medium being 10 to 30, the amino acid content in nitrogen source being 45 to 95%, the initial feed rate being in the range of 0 ml/hr to 12 ml/hr and being raised exponentially by an exponent in the range of 0.1 to 0.4 and/or linearly with the slope of the curve in the range of 0.5 to 3; and

b) inducing production with 0.01 - 4% inducer at a cell density of $OD_{600}$ 0.1 - $OD_{600}$ 50, feeding of the cell culture with the feed medium and feed rate of step (a) being continued after production has been induced and $pO_2$ being adjusted between 10% to 60% by passing sterile air into the fermentation broth and the temperature and pH of the fermentation broth being maintained at 33°C - 41°C and 6.9 - 8.5, respectively during the entire fermentation.

The host microorganisms used in the fermentation process of the invention may be Escherichia coli, strains of E-Coli such as JM109, W3110, TOP10 or BL21 and derivatives thereof and other enteric group bacteria such as Salmonella sp or Enterobactor sp and other eubacteria such as Bacillus sp, Pseudomonas sp or any other bacterium with similar growth requirements.

The vector used in the fermentation may be plasmid, pET or pBAD, pTOPO or any other commercial inducible vector.

The inducible promoters used in the fermentation may be T7 polymerase, uspA or araBAD or any other promoter present in commercial vectors.

The proteins produced or expressed by the fermentation process of the invention may be colony stimulating factors (CSFs) eg G-CSFs, growth hormones including human growth hormone (hGH), platelet derived growth factor (PDGF), β-galactosidase, interleukins such as IL-2 or IL-12 or tissue plasminogen activator (tPA) or any other recombinant peptide product. These proteins and their biological activities are known and reported.

The preferred proteins in this invention are recombinant human granulocyte colony stimulating factor (rhG-CSF), recombinant human growth hormone (rhGH), recombinant human Platelet Derived Growth factor (rhPDGF) or β-galactosidase.

Preferably, the feeding of the cell culture with the feed medium is after an $OD_{600}$ of 0.15 to 4 has been achieved or after 1 to 2 hrs from the start of the fermentation process.

Preferably the feed medium comprises 10 to 30% carbon source and 10 to 30% nitrogen source.

Preferably the feed medium comprises 50 to 400 mg of antibiotics and 3 to 4.25 gm of inorganic phosphates and trace elements.

The feed medium may comprise antibiotics such as ampicillin or tetracycline and inorganic phosphates and trace elements such as salts of calcium, potassium, magnesium, molybednum, boron, cobalt, manganese, iron, copper or zinc. Preferably the antibiotics is ampicillin and inorganic phosphates and trace elements comprise salts of iron, zinc, cobalt, boron, copper or calcium.

The carbon source may comprise glucose, glycerol, sorbitol, maltose, sucrose or starch. Preferably the carbon source is glucose or glycerol or mixture of glucose and glycerol.

The nitrogen source may comprise ammonia, nitrate, peptone, soya peptone, yeast extract, casamino acids or tryptone. Preferably the nitrogen source is yeast extract or soya peptone or tryptone or mixtures thereof.

Preferably, the amino acid content of the nitrogen source is in the range of 55% to 73% w/v.

Preferably, the expression of the heterologous proteins is induced at a cell density of $OD_{600}$ between 10 to 20.

Preferably the production is induced with 0.01% to 2% inducer.

The inducer may be IPTG, lactose, arabinose or maltose. Preferably the inducer is arabinose.

Preferably the pO2 is adjusted between 20%- 60%.

Preferably the temperature of the fermentation broth is maintained at 37°C.

Preferably pH of the fermentation broth is maintained at pH 6.9 - pH 7.5 and still preferably at pH 6.95 - pH 8.

The fermentation may be carried out for a period of 10 to 24 hours.

The yield of the heterologus proteins is at least 3 gms/L.

Preferably, the initial feed rate is in the range of 3 ml/hr to 12 ml/hr.

The feed rate may be increased exponentially by an exponent in the range of 0.1 to 0.4 using the formula

$$F=F_0 e^{kt}$$

where

 F=Feed rate at a time t (ml / hr)
 $F_o$ = Initial feed rate (ml / hr)
 k = exponent signifying a frequency of addition
 e = numerical constant equal to 2.718
 t = age of culture in hrs.

Preferably the exponent is in the range of 0.15 to 0.35. Still preferably the exponent is in the range of 0.2 to 4.3

The feed rate may be raised linearly with a slope of the curve in the range of 0.5 to 3 using the formula

$$y=mx + C$$

where

 y= Feed rate at a time t (ml / hr)
 x= Age of culture in hours
 m= Slope signifying total feed
 C= Initial feed rate (ml / hr)

Preferably the slope of the curve is in the range of 0.75 to 2.8. Still preferably the slope of the curve is in the range of 1.2 to 2.4.

The feed rate also may be increased by any polynomial function and/or linear combinations of any polynomial function(s).

The following experimental examples are illustrative of the invention but not limitative of the scope thereof :

The fermenters used in the examples were simple fermenters equipped with control devices comprising of temperature, pH and oxygen sensors and pumps for addition of feed medium, oxygen, acid/base, inducer and antifoam solutions. Construction of plasmid and transformation of host cells is done by known methods. The proteins may be recovered and purified by methods well known in the art.

The basal medium, used for the fermentation process, contained the following solutions BS1, BS2, BS3 and BS4:

BS1 was prepared by dissolving 2.5 - 15g of carbon source such as glucose or glycerol and 2.5 - 15g of nitrogen source such as yeast extract or soya peptone in 500 - 700 mL of RO water.

BS2 was prepared by dissolving 0.5 - 4g of ammonium sulphate, 0.8 - 3.2g of $KH_2PO_4$ and 3.3 - 13.2g of $Na_2HPO_4$. $2H_2O$ and 0.45 - 1.8g of NaCl in 50 - 200mL of RO water.

BS3 was prepared by dissolving 61.625 - 246.5g of $MgSO_4.2H_2O$ in 1000 mL of RO water.

All the above solutions were subjected to 20 - 40 minutes of autoclaving.
BS4 was 50mg/mL stock ampicillin solution sterilized through a 0.22 μ filter and stored at 4°C.
The basal medium was prepared as follows:
500 - 700 mL of BS1 was mixed with 100 - 1000 μL of antifoam solution (DOW Corning 1510, Antifoam or from HiMedia or Fluka), prior to autoclaving. To this solution a mixture of 50 - 200 mL of BS2 and 1 - 2 mL of BS3 was added to form the basal medium. The concentration of carbon source and nitrogen source in the basal medium was between p.25 - 1.5% w/v and 0.25 - 1.5% w/v, respectively.
The feed medium, used for the fermentation process, contained the following solutions.
FS 1, FS2, FS3 and FS4.
FS 1 was prepared by dissolving 60 - 180 g of carbon source such as glucose or glycerol in 175 - 200 mL of RO water.
FS2 was prepared by dissolving 60 - 180 g of nitrogen source such as yeast extract or soya peptone in 250 - 300 mL of RO water.
FS3 was prepared by dissolving 4.5 - 8 g of $KH_2PO_4$ and 6 - 9 g of $Na_2HPO_4$, $2H_2O$ in 25 - 50 mL of RO water.
All the above solutions were subjected to 20 - 40 minutes of autoclaving.
FS4 was prepared by dissolving 0.31 - 1.24 g of $H_3BO_3$, 0.088 - 0.322 g of $CoCl_2.6H_2O$, 0.025 - 0.1 g of $NaMoO_4.2H_2O$, 0.088 - 0.352 g of $CaCl_2.2H_2O$, 0.125 - 0.5 g of $MnSO_4$ $H_2O$, 2.1 - 8.35 g of $Fecl_3$ and 0.0125 - 0.05 g of $CuSO_{4.5}H_2O$ and 0.05 - 0.2 g of $ZnSO_{4.7}H_2O$, in 500 mL of RO water. The solution was filter sterilized through a 0.22 μ filter.
The feed medium was prepared by mixing 175 - 200 mL of FSI, 250 - 300 mL of FS2 and 25 - 250 mL of FS3. The concentration of carbon source, nitrogen source and inorganic phosphates in the feed medium was 10 - 30% w/v, 10 - 30% w/v and 2.5 - 4.25 w/v, respectively.

Example 1

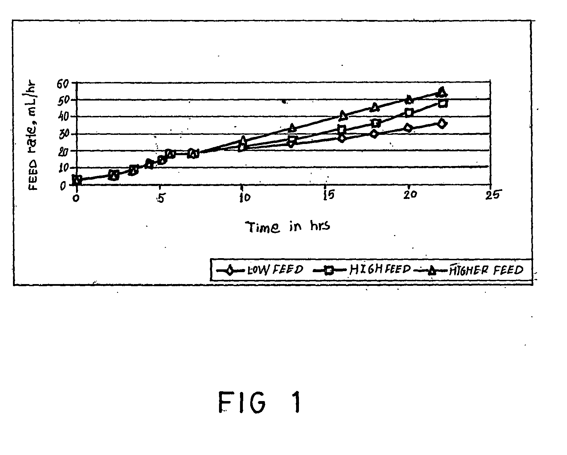

E Coli strain TOP10 transformed to express recombinant human G-CSF was purified and maintained in glycerol stocks. An aliquot of the culture was removed from the stock and streaked on 2XYT plate to separate single colonies after growth of 24 hours at 37°C. A single colony from the 2XYT plate was removed and inoculated into a falcon tube containing 10 ml of 2XYT liquid medium. After growth for 16 hours at 37°C on a rotary shaker (200 - 220 rpm), 5 ml of the culture from the tube was inoculated into a 500 ml conical flask containing 100 ml of the basal medium. After growth for 8 hours at 37°C on a rotary shaker (200 - 220 rpm), 100 mls of the culture from the flask was used to innoculate 2 jar fermenters (2 litres, B Braun) containing 900 ml of the basal medium.
Fermentation was carried out in the fermenters at a temperature of 37°C and pH of the two fermentation broths were maintained at pH7 using 12.5% of ammonia solution. The dissolved oxygen tension (dOT) was 50% air pressure for 16 hours of the fermentation time and was controlled by automatic adjustment of the fermenters stirrer speed. When $OD_{600}$ of approximately 1 was reached or at 2 hours after fermentation was started, the feed medium comprising glucose 25% (carbon source) and yeast extract 20% (nitrogen source) and further comprising FS4 solution (15 ml of FS4 solution for 600 ml of the feed medium) was pumped into the two fermenters following a feed strategy as set out in Fig 1 of the accompanying drawings (low feed rate). The initial feed rate was 3 ml/hr and was raised exponentially by an exponent of 0.3. After 8 hours from start of the fermentation or when a cell concentration of $OD_{600}$ 15 was obtained, one of the fermenters was inoculated with an inducer solution containing 0.1 - 4% inducer namely arabinose between $OD_{600}$ of 15 to 40. Excessive foaming was controlled with the addition of antifoam solution (Dow Corning 1510, Antifoam). Fermentations were performed for 25 hours and during that time samples were taken for measurement of optical density and accumulation of rhG-CSF within the cells. rhG-CSF accumulation was measured by scanning Coomassie stained SDS-PAGE gels of whole cell lysates in known manner.

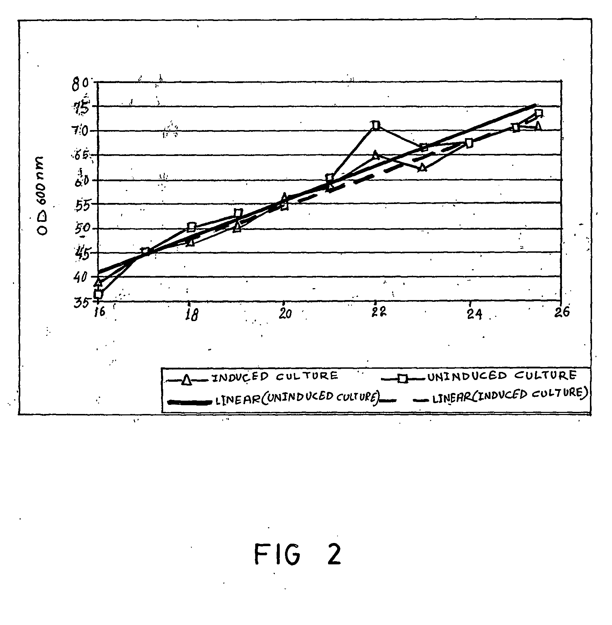

Yield of approximately 36% of rhG-CSF was obtained in the fermenter where production was induced. The experimental results are tabulated in Table 1 below and illustrated in Fig 2 of the accompanying drawings.

Table 1

| Growth of host E coli cells and expression of rhG-CSF during fermentation process of Example 1. | | | | |
|---|---|---|---|---|
| **Age of culture in the fermenter in hrs** | **Cell concentration by measuring OD at 600 nm** | | **Yield of rh G-CSF (% of total population)** | |
| | **Fermenter wherein production was induced after 8 hours from the start of the fermentation** | **Fermenter wherein production was not induced** | **Fermenter wherein production was induced after 8 hours from the start of the fermentation** | **Fermenter wherein production was not induced** |
| 0 | 0.17 | 0.175 | NE[a] | NE |
| 16 | 38.8 | 36.2 | 21.71 | NE |
| 17 | 45 | 45 | 18.76 | NE |
| 18 | 47.2 | 50.2 | 23.93 | NE |
| 19 | 50 | 52.8 | 21.60 | NE |
| 20 | 56.2 | 54.6 | 22.04 | NE |
| 21 | 58.6 | 59.6 | 30.77 | NE |
| 22 | 64.6 | 71 | 24.13 | NE |
| 23 | 62 | 66.4 | 33.1 | NE |
| 24 | 67.2 | 67.6 | 30.57 | NE |
| 25 | 71 | 70.6 | 35.87 | NE |
| 25.5 | 71 | 73.4 | 32 | NE |
| NE[α] No expression | | | | |

The experimental results show that the increase in bacterial density in the two fermenters is almost similar inspite of production being induced in one of the fermenters. A pronounced growth phase independent production of the protein rhG-CSF was observed. The cells grow at a constant rate throughout the entire fermentation process. Figure 2 illustrates the chronological course of the cell concentrations in the two fermenters wherein production of protein rhG-CSF was induced in one of the fermenters. Fig 2 shows the cell concentrations of the host E-Coli cells (measured at $OD_{600}$) as a function of time.

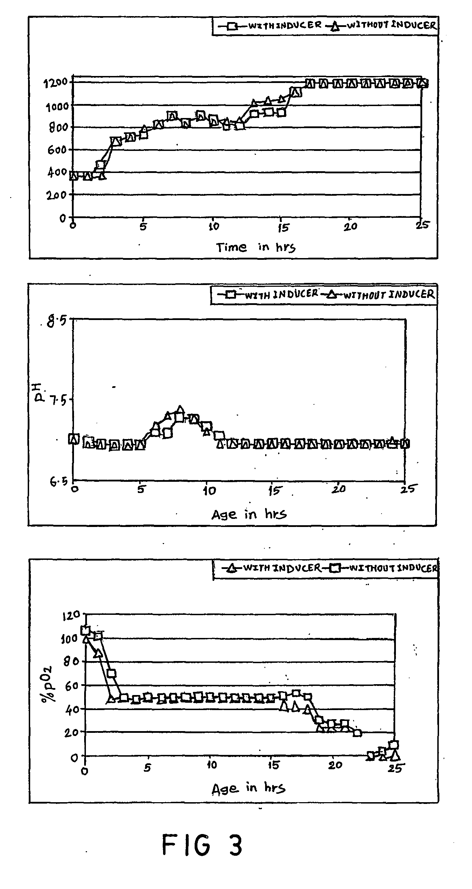

Fig 3 of the accompanying drawings shows stirrer speeds of the fermenters, pH of the fermentation broths and dissolved oxygen tensions maintained in the fermenters as functions of time. It is seen from the figure that except for the inoculation of one fermenter with the inducer solution, the feed rate, medium and fermentation conditions for the two fermenters were identical.

Example 1.1

E Coli strain TOP 10 transformed to express recombinant human G-CSF was purified and maintained in glycerol stocks. An aliquot of the culture was removed from the stock and streaked on 2XYT plate to separate single colonies after growth of 24 hours at 37°C. A single colony from the 2XYT plate was removed and inoculated into a falcon tube containing 10 ml of 2XYT liquid medium. After growth for 16 hours at 37°C on a rotary shaker (200 - 220 rpm), 5 ml of the culture from the tube was inoculated into a 500 ml conical flask containing 100 ml of the basal medium. After growth for 8 hours at 37°C on a rotary shaker (200 - 220 rpm), 100 ml of the culture from the flask was used to innoculate a jar fermenter (2 litres, B Braun) containing 900 ml of the basal medium.

Fermentation was carried out in the fermenter at a temperature of 37°C and pH of the fermentation broth was maintained at pH7 using 12.5% of ammonia solution. The dissolved oxygen tension (dOT) was 50% air pressure for 16 hours of the fermentation time and was controlled by automatic adjustment of the fermenter stirrer speed. When $OD_{600}$ of approximately 1 was reached or at 2 hours after fermentation was started, the feed medium comprising glucose 25% (carbon source) and yeast extract 20% (nitrogen source) and further comprising FS4 solution (15 ml of FS4 solution for 600 ml of the feed medium) was pumped into the fermenter following a feed strategy as set out in Fig 1 of the accompanying drawings (low feed rate). The initial feed rate was 3 ml/hr and was raised exponentially by an exponent of 0.3. After 8 hours from start of the fermentation or when a cell concentration of $OD_{600}$ 15 was obtained, the fermenter was inoculated with an inducer solution containing 0.1- 4% inducer namely arabinose between $OD_{600}$ of 15 to 40. Excessive foaming was controlled with the addition of antifoam solution (Dow Corning 1510, Antifoam). Fermentation was performed for 25 hours. During the fermentation process samples were taken for measurement of concentration of acetate, $NH_4^+$, $Na^+$ and $K^+$. The concentrations were measured using a Bioprofile 300 B Nova Bio analyzer. The experimental results are tabulated in Table 2 below.

Table 2

| The concentration of acetate $NH_4^+$, $Na^+$ and $K^+$ in the fermentation broth at various times of the fermentation process and at various cell concentrations measured at $OD_{600}$. | | | | | | |
|---|---|---|---|---|---|---|
| Age of culture in the fermenter in hrs | Cell concentration by measuring OD at 600 nm in the fermenter in which production was induced after 8 hours from the start of the fermentation | Acetate formed (g/L) in the fermenter | $NH_4$ + formed (g/L) in the fermenter | $Na^+$ formed (mmol/ L) in the fermenter | $K^+$ formed (mmol/L) in the fermenter | Yield of rh GCSF (% of total population) in the fermenter |
| 0 | 0.179 | 0.4 | 0.369 | 71 | 11.4 | NE [a] |
| 15 | 35.2 | 0.56 | 0.037 | 94 | 34.8 | 14.8 |
| 16 | 36.2 | 0.501 | 0.025 | 95 | 37.4 | 15 |
| 17 | 42.6 | 0.466 | 0.014 | 94 | 37.8 | 21 .5 |
| 18 | 44.8 | 0.537 | 0.009 | 95 | 40.7 | 22.3 |
| 19 | 46.4 | 0.72 | 0.01 | 95 | 41.5 | 24.8 |
| 20 | 48.4 | 0.56 | 0.012 | 95 | 43.8 | 29.3 |
| 21 | 52.2 | 0.46 | 0.015 | 95 | 46.5 | 31.5 |
| 22 | 59.4 | 0.84 | 0.023 | 94 | 47.4 | 31 |
| NE[a] No expression | | | | | | |

It is seen from the table that growth is not inhibited since formation of acetate is maintained at less than 1 gm/L for the duration of the fermentation process under the fermentation conditions. Therefore, the cells grow at a constant rate during the entire fermentation process resulting in good yield of the desired proteins rhG-CSF.

The following examples namely Examples 1a, 1b and 1c illustrate that by controlling the fermentation of Example 1 or Example 1.1 by

1) decreasing the concentration of carbon source in the feed medium
2) changing the rate at which cultures are fed with the feed medium and
3) changing the time at which production was induced higher expression or yield of the desired protein ie rhG-CSF was obtained.

Example 1a

E Coli strain TOP10 transformed to express recombinant human G-CSF was purified and maintained in glycerol stocks. An aliquot of the culture was removed from the stock and streaked on 2XYT plate to separate single colonies after growth of 24 hours at 37°C. A single colony from the 2XYT plate was removed and inoculated into a falcon tube containing 10 ml of 2XYT liquid medium. After growth for 16 hours at 37°C on a rotary shaker (200 - 220 rpm), 5 ml of the culture from the tube was inoculated into a 500 ml conical flask containing 100 ml of the basal medium. After growth for 8 hours at 37°C on a rotary shaker (200 - 220 rpm), 100 ml of the culture from the flask was used to inoculate a jar fermenter (2 litres, Braun) containing 900 ml of the basal medium. The fermentation conditions and duration of fermentation were the same as those in Example 1. The feed medium pumped into the fermenter was identical to that used in Example 1 except that the concentration of glucose (carbon source) in the feed medium was 20%. The feed strategy followed was also the same as that followed in Example 1. Production in the fermenter was induced as per Example 1. Yield of approximately 39 % of rhG-CSF was obtained.

Bacterial density achieved and percentage of rhG-CSF expressed in the fermenter was compared with the bacterial density achieved and percentage of rhG-CSF expressed in the fermenter of Example 1 wherein production was induced. The experimental results are tabulated in the Table 3 below:

Table 3

| Age of culture in the fermeters in hrs | Cell Concentration by measuring OD at 600 nm | | Yield of rhG-CSF (% of total population) | |
|---|---|---|---|---|
| | Fermenter of example 1 wherein production was induced and wherein glucose concentration of the feed medium was 25% | Fermenter of Example 1a wherein produced was induced and wherein glucose concentration of the feed medium was 20% | Fermenter of example 1 wherein production was induced and wherein glucose concentration of the feed medium was 25% | Fermenter of Example 1a wherein produced was induced and wherein glucose concentration of the feed medium was 20% |
| 0 | 0.183 | 0.259 | NE$^\alpha$ | 2.5 |
| 16 | 38.8 | 38.8 | 21.71 | 27.7 |
| 17 | 45 | 44.6 | 18.76 | 29.6 |
| 18 | 47.2 | 48 | 23.93 | 32.5 |
| 19 | 50 | 44.4 | 21.6 | 32.6 |
| 20 | 56.2 | 49 | 22.04 | 37.7 |
| 21 | 58.6 | 62.2 | 30.77 | 39 |
| NE [a] No expression | | | | |

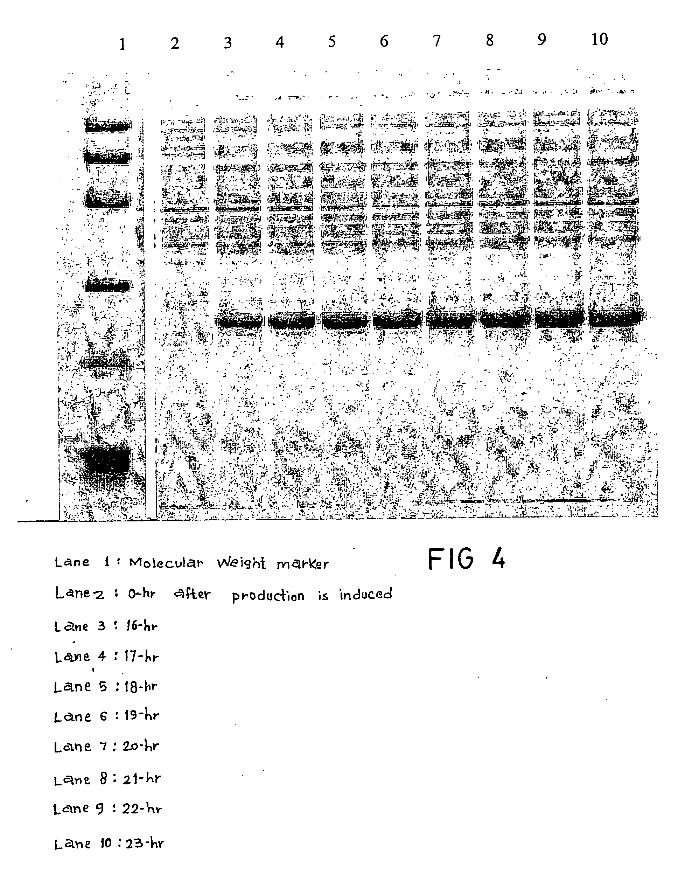

It is seen from the table that reduction in the concentration of glucose (carbon source) in the feed medium led to an increased yield or expression of the desired protein rhG-CSF by approximately 25% (Figure 4 of the accompanying drawings). Fig 4 shows SDS-PAGE analysis of culture samples of the fermentation process of Example 1a for accumulation of rhG-CSF. Lanes 1 - 9 correspond to the culture samples that were withdrawn from the fermentation broth of Example 1a at the hours in Table 3.

Example 1b

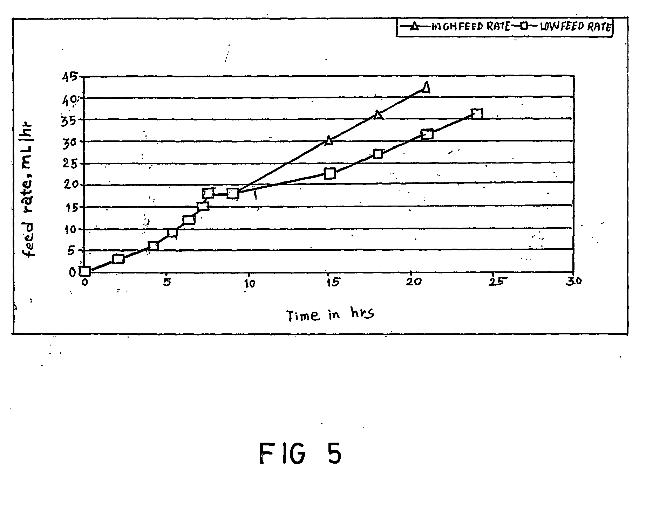

E Coli strain TOP 10 transformed to express recombinant human G-CSF was purified and maintained in glycerol stocks. An aliquot of the culture was removed from the stock and streaked on 2XYT plate to separate single colonies after growth of 24 hours at 37°C. A single colony from the 2XYT plate was removed and inoculated into a falcon tube containing 10 ml of 2XYT liquid medium. After growth for 16 hours at 37°C on a rotary shaker (200 - 220 rpm), 5 ml of the culture from the tube was inoculated into a 500 ml conical flask containing 100 ml of the basal medium. After growth for 8 hours at 37°C on a rotary shaker (200 - 220 rpm), 100 mls of the culture from the flask was used to inoculate 2 jar fermenters (2 litres, B Braun) containing 900 ml of the basal medium. The duration of fermentation was the same as that in Example 1. The feed medium pumped into the fermenters was identical to the one used in Example 1 and followed the feed strategies as set out in Fig 5 of the accompanying drawings. The initial feed rate was 3 ml/hr and was raised exponentially by an exponent of 0.3 till the feed rate reached 18 ml/hr. Thereafter, the feed rate was raised linearly with a slope of 1.2 (low feed rate). The initial feed rate was 3 ml/hr and was raised exponentially by an exponent of 0.3 till the feed rate reached 18 ml/hr. Then the feed rate was raised linearly with a slope of 2 (high feed rate). Production in the fermenters was induced as per Example 1. Yield of 27.23% rhG-CSF was obtained in the fermenter employing high feed rate and yield of 37.8% of rhG-CSF was obtained at approximately 21 hrs in the fermenter employing low feed rate.

Bacterial density achieved and percentage of rhG-CSF expressed in fermenter employing high feed rate (Fig 5) was compared with the bacterial density achieved and percentage of rhG-CSF expressed in fermenter employing low feed rate (Fig 5). The experimental results are tabulated in the Table 4 below :

Table 4

| Age of the culture in the fermenters in hrs | Cell concentration by measuring OD at 600 | | Yield of rhGCSF (% of total population) | |
|---|---|---|---|---|
| | Fermenter employing high feed rate | Fermenter employing low feed rate | Fermenter employing high feed rate | Fermenter employing low feed rate |
| 0 | 0.188 | 0.192 | NE[a] | NE |
| 15 | 42.4 | 35.8 | 19.408 | 25.99 |
| 16 | 46.2 | 35.4 | 21.868 | 30.1 |
| 17 | 51.6 | 38.4 | 21.05 | 33.3 |
| 18 | 56.5 | 41.2 | 23.019 | 36.7 |
| 19 | 62.2 | 47 | 25.382 | 37.8 |
| 20 | 59.4 | 49.4 | 26.029 | 38.2 |
| 20.5 | 55.2 | NS[b] | 27.231 | NS |
| 21 | NS | 50.8 | NS | 37.8 |
| 22 | NS | 57.8 | NS | 37.8 |
| 23 | NS | 55.6 | NS | 39.4 |

NE[a] No expression
NS[b] Not sampled

It is seen from the table that reduction in the rate at which feed medium is pumped into the fermenter led to an increased yield of the desired protein rhG-CSF by approximately 50% and did not inhibit the growth of the cells.

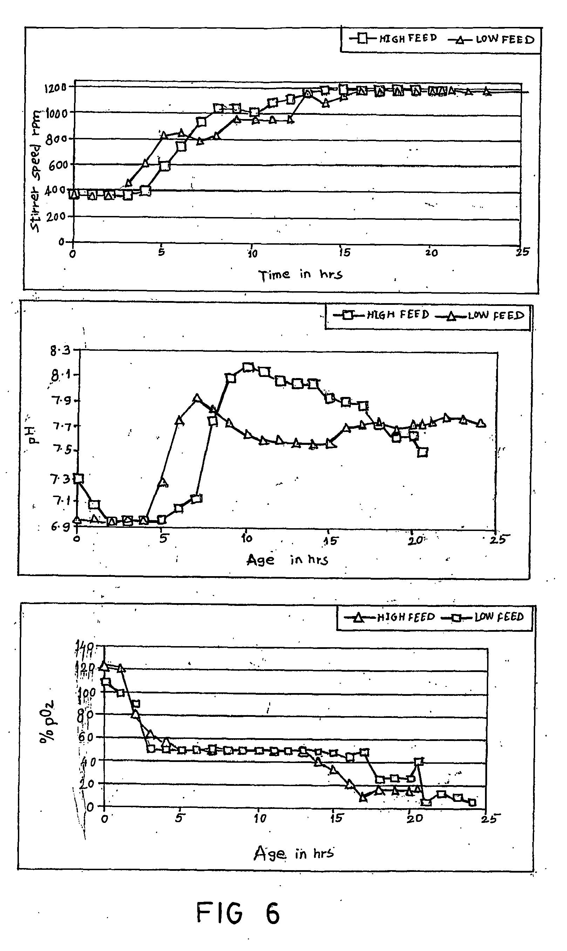

Figure 6 of the accompanying drawings illustrates the chronological course of stirrer speed, pH and dOT during the fermentation of Example 1b as functions of time. A comparison of the fermentation conditions (pH, stirrer speed and pO2) in the two fermenters shows that stirrer speed of the fermenter wherein feed medium was fed in at a low rate was less as compared to the stirrer speed of the other fermenter (Fig 6). Also, variations in pH and pO2 of the fermentation broth in the fermenter employing low feed rate is minimal. This shows that stresses on the cells in the fermenter employing low feed rate is less as compared to that in the fermenter employing high feed rate.

Example 1c

E Coli strain TOP 10 transformed to express recombinant human G-CSF was purified and maintained in glycerol stocks. An aliquot of the culture was removed from the stock and streaked on 2XYT plate to separate single colonies after growth of 24 hours at 37°C. A single colony from the 2XYT plate was removed and inoculated into a falcon tube containing 10 ml of 2XYT liquid medium. After growth for 16 hours at 37°C on a rotary shaker (200 - 220 rpm), 5 ml of the culture from the tube was inoculated into a 500 ml conical flask containing 100 ml of the basal medium. After growth for 8 hours at 37°C on a rotary shaker (200 - 220 rpm), 100 ml of the culture from the flask was used to inoculate two jar fermenters (2 litres, B Braun) containing 900 ml of the basal medium. The fermentation conditions and duration of fermentation were the same as those in Example 1. The feed medium and feed strategy were the same as those in Example 1a. Production of one fermenter was induced as per Example 1 ie after 8 hours from the start of the fermentation process. Production of the other fermenter was induced at the start of the fermentation process . Yield of approximately 39% of rhG-CSF was obtained in both the fermenters.

Bacterial density achieved and percentage of rhG-CSF expressed in two fermenters were compared. The experimental results were tabulated in the Table 5 below:

Table 5

| Age of culture in the fermenters in hrs | Cell concentration by measuring OD at 600 nm | | Yield of rhG-CSF (% of total population) | |
|---|---|---|---|---|
| | Fermenter in which production was induced after 8 hrs from the start of the fermentation | Fermenter in which production was induced at the start of the fermentation | Fermenter in which production was induced after 8 hrs from the start of the fermentation | Fermenter in which production was induced at the start of the fermentation |
| 0 | 0.259 | 0.195 | 2.5 | 3.08 |
| 16 | 38.8 | 39.8. | 27.7 | 26.7 |
| 17 | 44.6 | 44.2 | 29.6 | 30.9 |
| 18 | 48 | 46.6 | 32.5 | 32.3 |
| 19 | 44.4 | 59.4 | 32.6 | 30.7 |
| 20 | 49 | 55.2 | 37.7 | 36.4 |
| 21 | 62.2 | 56.4 | 39 | 37.1 |
| 22 | 62.2 | 58.8 | 38.1 | 39.5 |

It is seen from the table that the amount of rhG-CSF obtained in the fermenter wherein production was induced at the start of the fermentation was comparable to the amount of rhG-CSF obtained from the other fermenter wherein production was induced at a later point of time in the fermentation.

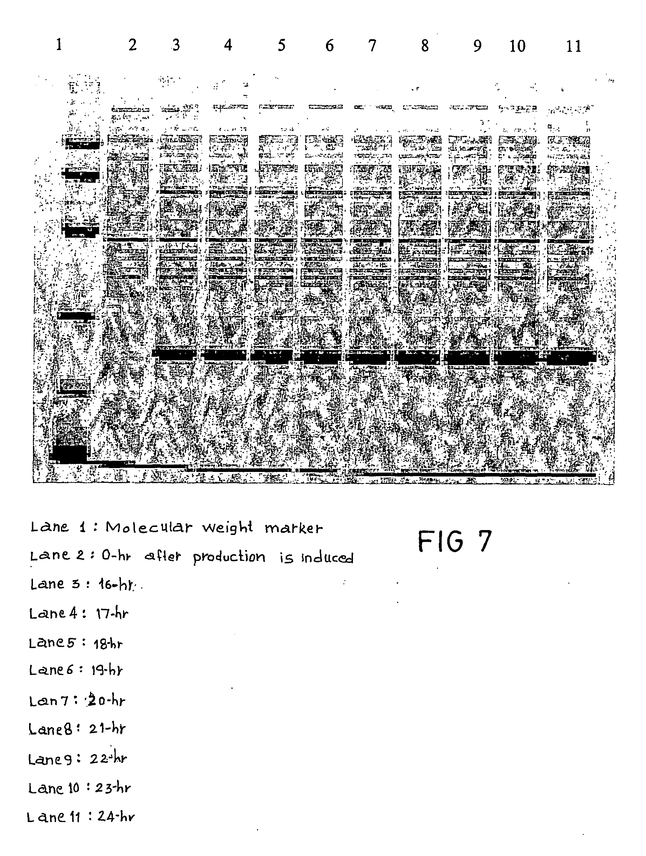

Also, cell concentrations achieved in the two fermenters were also similar ie production does not inhibit cell growth. This further establishes that cell growth and production of desired proteins are independent of each other. Fig 7 of the accompanying drawings shows SDS-PAGE analysis of culture samples of the fermentation process of Example 1c for accumulation of rhG-CSF. Lanes 1 - 9 correspond to the culture samples that were withdrawn from the fermentation broth of Example 1c at the time intervals in Table 5.

Example 2

E Coli strain TOP 10 transformed to express recombinant human growth hormone (rhGH) was purified and maintained in glycerol stocks. An aliquot of the culture was removed from stock and streaked on 2XYT plate to separate single colonies after growth of 24 hours at 37°C. A single colony from the 2XYT plate was removed and inoculated into a falcon tube containing 10 ml of 2XYT liquid medium. After growth for 16 hours at 37°C on a rotary shaker (200 - 220 rpm), 5 ml of the culture from the tube was inoculated into a 500 ml conical flask containing 100 ml of the basal medium. After growth for 8 hours at 37°C on a rotary shaker (200 - 220 rpm), 100 mls of the culture from the flask was used to innoculate 2 jar fermenters (2 litres, B Braun) containing 900 ml of the basal medium.

The two fermentations were carried out at a temperature of 37°C and pH of the two fermentation broths were maintained at pH7 using 12.5% of ammonia solution. The dissolved oxygen tension (dOT) was 50% air pressure for 16 hours of the fermentation time and was controlled by automatic adjustment of the fermenters stirrer speed. When $OD_{600}$ of approximately 1 was reached or at 2 hours after fermentation was started, feed medium prepared as per the description furnished above and having concentration of glucose 25% (carbon source) and yeast extract 20% (nitrogen source) and comprising of FS4 solution (15 ml of FS4 solution for 600 ml of the feed medium) was pumped into the two fermenters following a feed strategy as set out in Fig 1 (high feed rate). The initial feed rate was 3 ml/hr and was raised exponentially till 18 mL/hr and thereafter was raised linearly with a slope of 2. After 8 hours from start of the fermentation or when a cell concentration of $OD_{600}$ 13 was obtained, one of the fermenters was inoculated with an inducer solution containing 0.1-4% inducer namely arabinose between $OD_{600}$ of 13 to 40. Excessive foaming was controlled with the addition of antifoam solution (Dow Corning 1510, Antifoam). Fermentations were performed for 23 hours and during that time samples were taken for measurement of optical density and accumulation of rhGH within the cells. rhGH accumulation was measured by scanning Coomassie stained SDS-PAGE gels of whole cell lysates in known manner.

Yield of approximately 24% of rhGH was obtained. The experimental results are tabulated in the Table 6 below :

Table 6

| Growth of E Coli cells and expression of rhGH during fermentation process of Example 2 | | | | |
|---|---|---|---|---|
| Age of culture in the fermenters in hrs | Cell concentration by measuring OD at 600 nm | | Yield of rhGH (% of total population) | |
| | Fermenter wherein production was induced after 8 hours from the start of the fermentation | Fermenter wherein production was not induced | Fermenter wherein production was induced after 8 hours from the start of the fermentation | Fermenter wherein production was not induced |
| 0 | 0.2 | 0.204 | NE$^a$ | NE |
| 17 | 41.2 | 42.4 | 22.08 | NE |
| 18 | 46.4 | 45.6 | 21.58 | NE |
| 19 | 44 | 44.4 | 22.48 | NE |
| 20 | 34.8 | 36 | 22.35 | NE |
| 21 | 49.8 | 56 | 24.78 | NE |
| 22 | 51.8 | 54.8 | 21.05 | NE |
| 23 | 55 | 58.2 | 22.05 | NE |
| NE$^a$ No expression | | | | |

The experimental results showed that the increase in bacterial density in the two fermenters was almost similar inspite of production being induced in one of the fermenters. A pronounced growth phase independent of production of the protein rhGH was observed. The cells grow at a constant rate throughout the entire fermentation process.

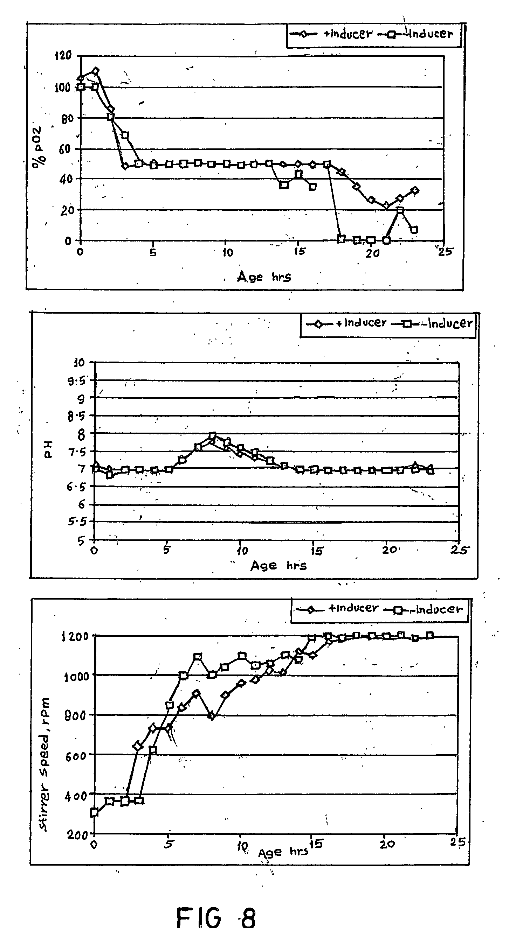

Figure 8 of the accompanying drawings illustrates the chronological course of stirrer speed, pH and dOT during the fermentation of Example 2 as functions of time. It is seen from the figure that except for the inoculation of one fermenter with the inducer solution, the feed rate medium and fermentation conditions for the two fermenters were identical.

Example 2.1

E Coli strain TOP 10 transformed to express recombinant human growth hormone (rhGH) was purified and maintained in glycerol stocks. An aliquot of the culture was removed from stock and streaked on 2XYT plate to separate single colonies after growth of 24 hours at 37°C. A single colony from the 2XYT plate was removed and inoculated into a falcon tube containing 10 ml of 2XYT liquid medium. After growth for 16 hours at 37°C on a rotary shaker (200 - 220 rpm), 5 ml of the culture from the tube was inoculated into a 500 ml conical flask containing 100 ml of the basal medium. After growth for 8 hours at 37°C on a rotary shaker (200 - 220 rpm), 100 ml of the culture from the flask was used to innoculate a jar fermenter (2 litres, B Braun) containing 900 ml of the basal medium.

The fermentation was carried out at a temperature of 37°C and pH of the fermentation broth was maintained at pH7 using 12.5% of ammonia solution. The dissolved oxygen tension (dOT) was 50% air pressure for 16 hours of the fermentation time and was controlled by automatic adjustment of the fermenter stirrer speed. When OD$_{600}$ of approximately 1 was reached or at 2 hours after fermentation was started, feed medium prepared as per the description furnished above and having concentration of glucose 25% (carbon source) and yeast extract 20% (nitrogen source) and comprising of FS4 solution (15 ml of FS4 solution for 600 ml of the feed medium) was pumped into the fermenter following a feed strategy as set out in Fig 1 (high feed rate).). The initial feed rate was 3 ml/hr and was raised exponentially till 18 mL/hr and thereafter was raised linearly with a slope of 2. After 8 hours from start of the fermentation or when a cell concentration of OD$_{600}$ 13 was obtained, the fermenter was inoculated with an inducer solution containing 0.1- 4% inducer namely

arabinose between $OD_{600}$ of 13 to 40. Excessive foaming was controlled with the addition of antifoam solution (Dow Corning 1510, Antifoam). Fermentation was performed for 23 hours. During the fermentation process samples were also taken for measurement of concentration of acetate, $NH_4^+$, $Na^+$ and $K^+$. The concentrations were measured using a Bioprofile 300 B Nova Bio analyzer. The experimental results are tabulate in the Table 7 below :

Table 7

| The concentration of acetate, $NH_4^+$, $Na^+$ , $K^+$ in the fermentation broth at various times of the fermentation process at various cell concentrations measured at $OD_{600}$ | | | | | | |
|---|---|---|---|---|---|---|
| Age of culture in the fermen -ters in hrs | Cell concentration by measuring OD at 600 nm in the fermenter in which production was induced after 8 hours from the start of the fermentation | Acetate formed (g/L) in the fermenter LCY | $NH_4^+$ formed (g/L) in the fermen -ter | $Na^+$ formed (mmol/ L) in the fermenter | $K^+$ formed (mmol/L) in the fermenter | Yield of rh GH (% of total population) in the fermenter |
| 0 | 0.206 | 0.36 | 0.385 | 60.1 | 12.8 | NE[a] |
| 15 | 38.6 | 1.2 | 0.315 | 68 | 37.8 | 20 |
| 16 | 37 | 1.11 | 0.283 | 68.8 | 39.6 | 19.3 |
| 17 | 43.2 | 1.03 | 0.272 | 70 | 42.2 | 18.93 |
| 18 | 42.4 | 0.94 | 0.241 | 70.4 | 44.4 | 19.94 |
| 19 | 48.4 | 0.86 | 0.214 | 70.7 | 47.4 | 23.23 |
| 20 | 51 | 0.92 | 0.211 | 71.2 | 53.5 | NS[b] |
| 21 | 50.2 | 1.01 | 0.198 | 72.1 | 55.9 | 23.74 |
| 22 | 52.8 | 1.07 | 0.211 | 72.8 | 60.1 | 22.07 |
| 23 | 52.4 | 1.31 | 0.317 | 73.4 | 61.9 | 23.48 |
| 24 | 50.8 | 2.06 | 0.523 | 74.7 | 65.8 | 24.78 |
| NE[a] No expression NS[b] Not sampled | | | | | | |

It is seen from the table that growth was not inhibited since formation of acetate was maintained at less than 2 gm/L for the duration of the fermentation under the fermentation conditions selected. Therefore, the cells grow at a constant rate during the entire fermentation resulting in good yield of the desired proteins rhGH.

Example 2a

E Coli strain TOP 10 transformed to express recombinant human growth hormone rhGH was purified and maintained in glycerol stocks. An aliquot of the culture was removed from the stock and streaked on 2XYT plate to separate single colonies after growth of 24 hours at 37°C. A single colony from the 2XYT plate was removed and inoculated into a falcon tube containing 10 ml of 2XYT liquid medium. After growth for 16 hours at 37°C on a rotary shaker (200 - 220 rpm), 5 ml of the culture from the tube was inoculated into a 500 ml conical flask containing 100 ml of the basal medium. After growth for 8 hours at 37°C on a rotary shaker (200 - 220 rpm), 100 ml of the culture from the flask was used to inoculate a jar fermenter (2 litres, Braun) containing 900 ml of the basal medium. The fermentation conditions and duration of fermentation were the same as those in Example 2. The feed medium pumped into the fermenter was identical to that used in Example 2 except that the carbon source in the feed medium was glycerol (25%). The feed strategy followed was also the same as that of Example 2. Production in the fermenter was induced as per Example 2. Yield of approximately 26% of rhGH was obtained. The experimental results were tabulated in the Table 8 below:'

Table 8

| Age of culture in the fermenter in hrs | Cell Concentration by measuring OD at 600 nm in the fermenter in which Production was induced after 8 hours from the start of fermentation | Yield of rh GH (% of total population) in the fermenter |
|---|---|---|
| 0 | 0.26 | NE[a] |
| 1 | 0.434 | NE |
| 2 | 1.324 | NE |
| 3 | 2.87 | NE |
| 4 | 6.6 | NE |
| 5 | 11.1 | NE |
| 6 | 12.9 | NE |
| 7 | 15.2 | NE |
| 8 | 18.5 | NE |
| 24 | 48.2 | 24 |
| 25 | 49.6 | 22.5 |
| 26 | 49.4 | 26 |
| NE[a] No expression | | |

The experimental results show that the increase in bacterial density and percentage of yield of rhGH were almost similar to those obtained in the fermenter of Example 2 wherein production was induced.

Example 2b

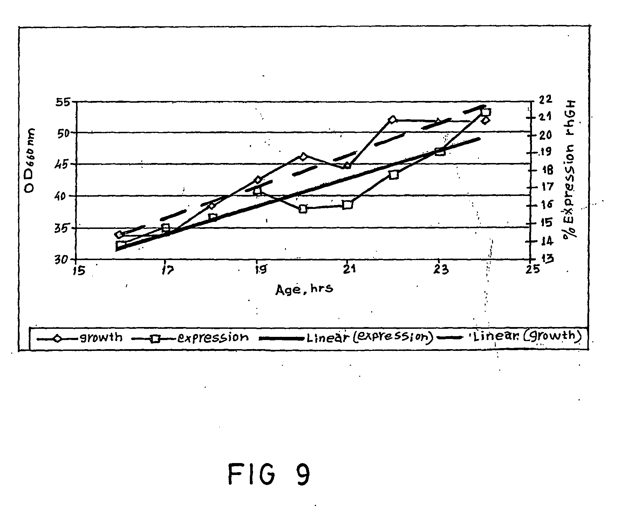

E Coli strain TOP10 transformed to express recombinant human growth hormone rhGH was purified and maintained in glycerol stocks. An aliquot of the culture was removed from the stock and streaked on 2XYT plate to separate single colonies after growth of 24 hours at 37°C. A single colony from the 2XYT plate was removed and inoculated into a falcon tube containing 10 ml of 2XYT liquid medium. After growth for 16 hours at 37°C on a rotary shaker (200 - 220 rpm), 5 ml of the culture from the tube was inoculated into a 500 ml conical flask containing 100 ml of the basal medium. After growth for 8 hours at 37°C on a rotary shaker (200 - 220 rpm), 100 ml of the culture from the flask was used to inoculate ajar fermenter (2 litres, Braun) containing 900 ml of the basal medium. The fermentation conditions and duration of fermentation were the same as those in Example 2. The feed medium pumped into the fermenter was identical to that used in Example 2 except that the nitrogen source in the feed medium was soya peptone (20%). The feed strategy followed was also the same as that of Example 2. Production in the fermenter was induced as per Example 2. Yield of approximately 24% of rhGH was obtained. The experimental results are tabulated in the Table 9 below and illustrated in Fig 9 of the accompanying drawings.

Table 9

| Age of culture in the fermenter in hrs | Cell Concentration by measuring OD at 600 nm in the fermenter in which Production was induced after 8 hrs from the start of the fermentation | Yield of rh GH (% of total population) in the fermenter |
|---|---|---|
| 0 | 0.233 | NE[a] |
| 16 | 33.8 | 13.83 |

(continued)

| Age of culture in the fermenter in hrs | Cell Concentration by measuring OD at 600 nm in the fermenter in which Production was induced after 8 hrs from the start of the fermentation | Yield of rh GH (% of total population) in the fermenter |
|---|---|---|
| 17 | 34 | 14.74 |
| 18 | 38.4 | 15.35 |
| 19 | 42.4 | 16.85 |
| 20 | 46.2 | 15.85 |
| 21 | 44.6 | 16.05 |
| 22 | 52 | 17.79 |
| 23 | 51.8 | 19.08 |
| 24 | 51.8 | 21.35 |
| NE[a] No expression | | |

Figure 9 illustrates the chronological course of the cell concentrations of the host E-coli cells (measured at $OD_{600}$) and percentage of expression of rhGH as functions of time in the fermenter of Example 2b.

The experimental results show that increase in bacterial density and percentage of yield of rhGH were almost similar to those, obtained in the fermenter of Example 2 wherein production was induced.

The following examples namely Examples 2c and 2d illustrate that controlling of the fermentation of Example 2 by

1) changing the rate at which cultures were fed with the feed medium and
2) changing the concentration of the inducer solution fed into the fermenter resulted in higher expression or yield of the desired protein ie rhGH.

Example 2c

E Coli strain TOP10 transformed to express recombinant human growth hormone rhGH was purified and maintained in glycerol stocks. An aliquot of the culture was removed from the stock and streaked on 2XYT plate to separate single colonies after growth of 24 hours at 37°C. A single colony from the 2XYT plate was removed and inoculated into a falcon tube containing 10 ml of 2XYT liquid medium. After growth for 16 hours at 37°C on a rotary shaker (200 - 220 rpm), 5 ml of the culture from the tube was inoculated into a 500 ml conical flask containing 100 ml of the basal medium, After growth for 8 hours at 37°C on a rotary shaker (200 - 220 rpm), 100 mls of the culture from the flask was used to inoculate 2 jar fermenters (2 litres, B Braun) containing 900 ml of the basal medium. The fermentation conditions and duration of fermentation were the same as those in Example 2. The feed medium pumped into the fermenters was identical to the one used in Example 2 and followed the feed strategies as set out in Fig 1 (high feed rate and higher feed rate). In the first fermenter, the initial feed rate was 3 ml/hr and was raised exponentially till 18 mL/hr and thereafter linearly with a slope of 2 (high feed rate). In the second fermenter, the initial feed rate was 3 ml/hr and was raised exponentially till 18 mL/hr and thereafter linearly with a slope of 2.4. (higher feed rate). Production in the fermenters was induced as per Example 2. Yield of 32% of rhGH was obtained in the fermenter employing high feed rate and 34% of rhGH was obtained in the fermenter employing higher feed rate.

Bacterial density achieved and percentage of rhGH expressed in fermenter employing high feed rate (Fig 1) was compared with the bacterial density achieved and percentage of rhGH expressed in the fermenter employing higher feed rate (Fig 1). The experimental results are tabulated in Table 10 below:

Table 10

| Age of culture in the fermenter in hrs | Cell concentration by measuring OD at 600 | | Yield of rhGH (% of total population) in the fermenter | |
|---|---|---|---|---|
| | Fermenter employing high feed rate | Fermenter employing higher feed rate | Fermenter employing high feed rate | Fermenter employing higher feed rate |
| 0 | 0.187 | NS [b] | NE[a] | NE |
| 16 | 38.2 | NS | 25.75 | NS |
| 17 | 44.4 | NS | 25.38 | NS |
| 18 | 46.4 | NS | 27.35 | NS |
| 19 | 47.6 | 59 | 30.23 | NS |
| 20 | 48.8 | 71 | 31.045 | NS |
| 21 | 47.4 | 71.8 | 31.89 | 33.83 |
| 22 | 54.6 | NS | 31.05 | NS |
| 23 | 58.4 | NS | 28.9 | NS |
| NE[a] No expression NS[b] Not sampled | | | | |

It is seen from the table that reduction in the rate at which feed medium was pumped into the fermenter led to an increased volumetric yield of the desired protein rhGH by approximately 45% and did not inhibit the growth of the cells.

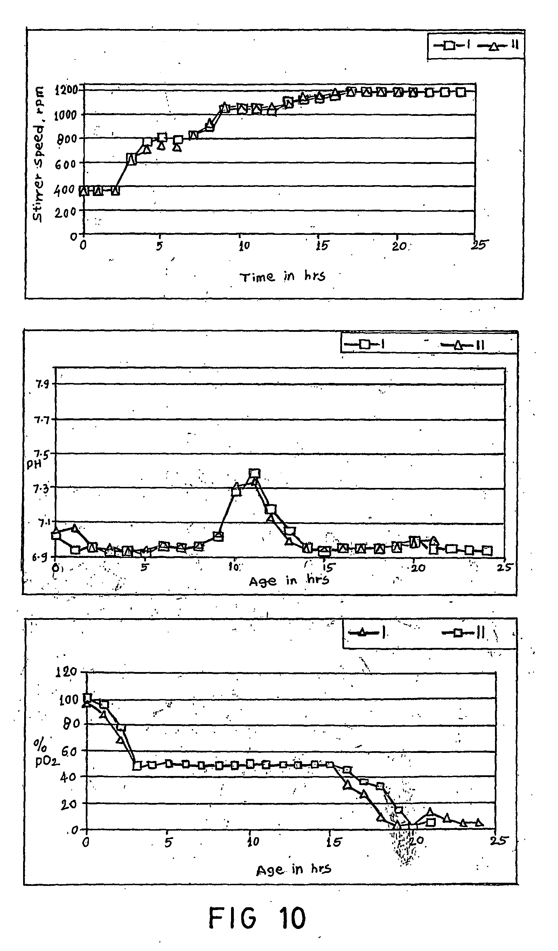

Figure 10 of the accompanying drawings illustrates the chronological course of stirrer speed, pH and dOT during the fermentation of Example 2c as functions of time. A comparison of the fermentation conditions (pH, stirrer speed and pO2) in the two fermenters seen in the figure shows that there are no variations, indicating that cell physiology remained the same in the two fermenters inspite of the difference in the feed rate. However, bacterial density achieved and percentage of rhGH expressed in the fermenter employing higher feed rate was more .

Example 2d

E Coli strain TOP 10 transformed to express recombinant human GH was purified and maintained in glycerol stocks. An aliquot of the culture was removed from the stock and streaked on 2XYT plate to separate single colonies after growth of 24 hours at 37°C. A single colony from the 2XYT plate was removed and inoculated into a falcon tube containing 10 ml of 2XYT liquid medium. After growth for 16 hours at 37°C on a rotary shaker (200 - 220 rpm), 5 ml of the culture from the tube was inoculated into a 500 ml conical flask containing 100 ml of the basal medium. After growth for 8 hours at 37°C on a rotary shaker (200 - 220 rpm), 100 ml of the culture from the flask was used to inoculate three jar fermenters (2 litres, B Braun) containing 900 ml of the basal medium. The fermentation conditions and duration of fermentation were the same as those in Example 2. The feed medium pumped into the fermenters and the feed strategy were the same as those in Example 2. Production in the fermenters was induced after 8 hours from the start of the fermentation by inoculation of the fermenters with inducer solutions containing arabinose. The concentrations of the inducer in the three fermenters was 0.1 %, 0.25% and 1%, respectively. Yields of 17%, 15% and 18% of rhGH over total protein were obtained in the fermenters corresponding to inducers of 0.1%, 0.25% and 1 % respectively.

Bacterial density achieved and percentage of rhGH expressed in the three fermenters were compared. The experimental results were tabulated in the Table 11 below:

Table 11

| Age of culture in the fermenters in hrs | Cell concentration by measuring OD at 600 nm | | | Yield of rhGH (% of total population) | | |
|---|---|---|---|---|---|---|
| | Fermenter in which production was induced by inducer solution wherein concentr -ation of the inducer was 1 % | Fermenter in which production was induced by inducer solution wherein concentration of the inducer was 0.25% | Fermenter in which production was induced by inducer solution wherein concentration of the inducer was 0.1 % | Fermenter in which production was induced by inducer solution wherein concentration of the inducer was 1% | Fermenter in which production was induced by inducer solution wherein concentration of the inducer was 0.25% | Fermenter in which production was induced by inducer solution wherein concentration of the inducer was 0.1% |
| 0 | 0.176 | 0.18 | 0.183 | NE$^\alpha$ | NE | NE |
| 19 | 36.6 | 36.4 | 36 | 13.23 | 15.15 | 17.5 |
| 20 | 37.2 | 38 | 41.4 | 13.23 | 15.69 | 16.2 |
| 21 | 35.2 | 34 | 41.6 | 15.05 | 12.31 | 13.96 |
| 22 | 38.8 | 36.4 | 43.4 | 17.5 | 12.58 | 14.02 |
| 23 | 37.4 | 34.2 | 42 | 18.66 | 8.88 | 10.775 |
| 24 | 38.2 | 33.8 | 45.4 | 18.67 | 9.15 | 10.5 |
| NE$^\alpha$ No expression | | | | | | |

The experimental results show that irrespective of the concentration of the inducer in the inducer solution percentage of expression remains the same except that high levels of expressions in the three fermenters occurred at different points of time of fermentation.

Example 3

E Coli strain TOP 10 transformed to express recombinant human platelet derived growth factor rhPDGF was purified and maintained in glycerol stocks. An aliquot of the culture was removed from the stock and streaked on 2XYT plate to separate single colonies after growth of 24 hours at 37°C. A single colony from the 2XYT plate was removed and inoculated into a falcon tube containing 10 ml of 2XYT liquid medium. After growth for 16 hours at 37°C on a rotary shaker (200 - 220 rpm), 5 ml of the culture from the tube was inoculated into a 500 ml conical flask containing 100 ml of the basal medium. After growth for 8 hours at 37°C on a rotary shaker (200 - 220 rpm), 100 mls of the culture from the flask was used to innoculate 2 jar fermenters (2 litres, B Braun) containing 900 ml of the basal medium.

The two fermentations were carried out at a temperature of 37°C and pH of the two fermentation broths were maintained at pH7 using 12.5% of ammonia solution. The dissolved oxygen tension (dOT) was 50% air pressure for 16 hours of the fermentation time and was controlled by automatic adjustment of the fermenters stirrer speed. When $OD_{600}$ of approximately 2 was reached or at 2 hours after fermentation was started, feed medium containing concentration of glucose 20% (carbon source) and yeast 20% (nitrogen source) and comprising FS4 solution (15 ml of FS4 solution for 600 ml of the feed medium) was pumped into the two fermenters following a feed strategy as set out in Fig 1 (low feed rate). The initial feed rate was 0.1 ml/hr and was raised exponentially till 18 mL/hr and thereafter linearly by a slope of 1.2. After 8 hours from the start of the fermentation or when a cell concentration of $OD_{600}$ 20 was obtained, one of the fermenters was inoculated with an inducer solution containing 0.1 - 4% of the inducer arabinose between $OD_{600}$ of 20 to 50. Excessive foaming was controlled with the addition of antifoam solution (Dow Corning 1510, Antifoam). Fermentations were performed for 24 hours during which samples were taken for measurement of optical density and accumulation of rhPDGF within the cells. rhPDGF accumulation was measured by scanning Coomassie stained SDS-PAGE gels of whole cell lysates in known manner.

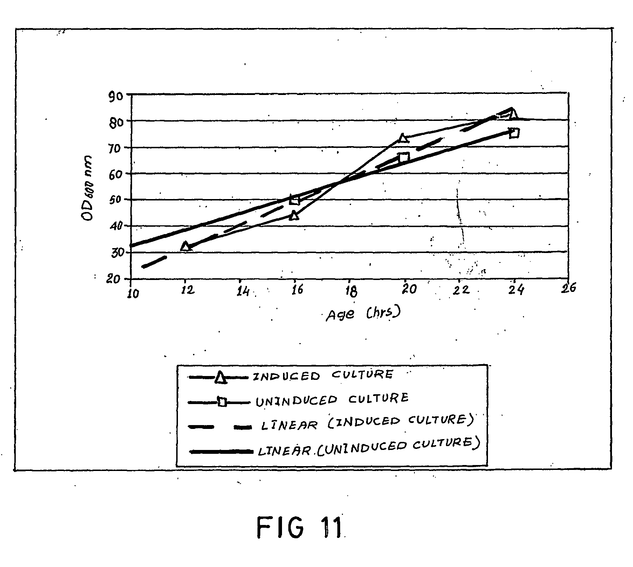

Yield of approximately 46% of rhPDGF was obtained. The experimental results are tabulated in the Table 12 below and illustrated in Fig 11 of the accompanying drawings.

Table 12

| Growth of host E coli cells and expression of rhPDGF during the fermentation of Example 3. | | | | |
|---|---|---|---|---|
| **Age of culture in the fermenters in hrs** | **Cell concentration by measuring OD at 600 nm** | | **Yield of rhPDGF (% of total population)** | |
| | **Fermenter in which production was induced after 8 hours from the start of the fermentation** | **Fermenter in which production was not induced** | **Fermenter in which production was induced after 8 hours from the start of the fermentation** | **Fermenter in which production was not induced** |
| 0 | 0.19 | 0.21 | NE[a] | NE |
| 12 | 32.4 | NS[b] | 25.56 | NE |
| 16 | 44.2 | 50.4 | 38.89 | NE |
| 20 | 73.2 | 65.6 | 42.3 | NE |
| 24 | 82.2 | 75.3 | 45.82 | NE |
| NE[α] No expression<br>NS[b] Not sampled | | | | |

The experimental results show that the increase in bacterial density in the two fermenters is almost similar inspite of production being induced in one of the fermenters. A pronounced growth phase independent of production of the protein rhPDGF was observed. The cells grow at a constant rate throughout the entire fermentation process.

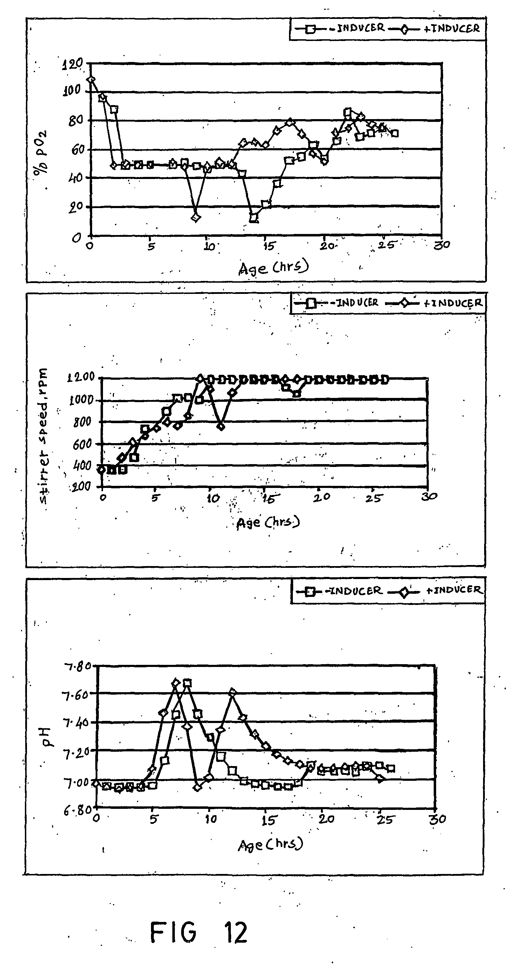

Fig 12 of the accompanying drawings illustrates the chronological course of stirrer speed, pH and dOT during the fermentation of Example 3 as functions of time. It is seen from the figure that except for the inoculation of one fermenter with the inducer solution, the feed rate medium and fermentation condition for the two fermenters were identical.

Example 3a

E Coli strain TOP10 transformed to express recombinant human platelet derived growth factor rhPDGF was purified and maintained in glycerol stocks. An aliquot of the culture was removed from stock and streaked on 2XYT plate to separate single colonies after growth of 24 hours at 37°C. A single colony from the 2XYT plate was removed and inoculated into a falcon tube containing 10 ml of 2XYT liquid medium. After growth for 16 hours at 37°C on a rotary shaker (200 - 220 rpm), 5 ml of the culture from the tube was inoculated into a 500 ml conical flask containing 100 ml of the basal medium. After growth for 8 hours at 37°C on a rotary shaker (200 - 220 rpm), 100 mls of the culture from the flask was used to innoculate 2 jar fermenters (2 litres, B Braun) containing 900 ml of the basal medium. The fermentation conditions and duration of fermentation were the same as those in Example 3. The feed strategy was the same as that in Example 3. The feed medium pumped into one of fermenters was same as that of Example 3 except that the concentration of glucose (carbon source) in the feed medium was 20% and basal medium contained 50 mg/L of ampicillin. The feed medium pumped into the other fermenter was the same as that fed into the first fermenter except that it did not comprise of ampicillin. Production in the fermenters was induced as per Example 3. Yield of 42% of rhPDGF was obtained in the fermenter with ampicillin and 51 % in the fermenter where ampicillin was not added.

Bacterial density achieved and the percentage of rhPGDF expressed in the two fermenters were compared. The experimental results were tabulated in the Table 13 below:

Table 13

| Age of culture in the fermenters in hrs | Cell concentration by measuring OD at 600 nm | | Yield of rhPDGF (% of total population) | |
|---|---|---|---|---|
| | Fermenter containing feed medium with ampicillin | Fermenter containing feed medium without ampicillin | Fermenter containing feed medium with ampicillin | Fermenter containing feed medium without ampicillin |
| 0 | 0.17 | 0.21 | NE[a] | NE |
| 8 | _[b] | 14.8 | NS[b] | NE |
| 16 | 52.6 | NS[b] | 35.29 | NS |
| 17 | 51.2 | NS | 38.07 | NS |
| 18 | 55.6 | NS | 37.77 | NS |
| 19 | 59 | NS | 39.03 | NS |
| 20 | 64 | NS | 40.13 | NS |
| 21 | 72.6 | NS | 39.57 | NS |
| 22 | 70 | NS | 39.84 | NS |
| 23 | 74 | 63 | 43.37 | 49.72 |
| 24 | 72.2 | NS | 42.41 | NS |
| 25 | NS | 70.2 | NS | 51.76 |
| NE[a] No expression NS[b] Not sampled | | | | |

It is seen from the table that inclusion of ampicillin (antibiotic) in the feed mediumn led to an increased yield or expression of the desired protein rhPDGF by approximately 22%.

The following examples namely Examples 3b and 3c illustrate that controlling of the fermentation of Example 3 by

1) increasing concentration of carbon source in the feed medium and

2) changing the concentration of the inducer solution fed into the fermenter resulted in higher expression or yield of the desired protein ie rhPDGF.

Example 3b

E Coli strain TOP10 transformed to express rhPDGF was purified and maintained in glycerol stocks. An aliquot of the culture was removed from the stock and streaked on 2XYT plate to separate single colonies after growth of 24 hours at 37°C. A single colony from the 2XYT plate was removed and inoculated into a falcon tube containing 10 ml of 2XYT liquid medium. After growth for 16 hours at 37°C on a rotary shaker (200 - 220 rpm), 5 ml of the culture from the tube was inoculated into a 500 ml conical flask containing 100 ml of the basal medium. After growth for 8 hours at 37°C on a rotary shaker (200 - 220 rpm), 100 ml of the culture from the flask was used to inoculate a jar fermenter (2 litres, Braun) containing 900 ml of the basal medium. The fermentation conditions and duration of fermentation were the same as those in Example 3. The feed medium pumped into the fermenter was identical to that used in Example 3 except that the concentration of glucose (carbon source) in the feed medium was 25%. The feed strategy followed was also the same as that followed in Example 3. Production in the fermenter was induced as per Example 3. Yield of approximately 40% rhPDGF (of total protein) was obtained.

Bacterial density achieved and percentage of rhPDGF expressed in the fermenter of this Example were compared with the bacterial density achieved and percentage of rhPDGF expressed in the fermenter of Example 3 wherein production was induced.

A comparative fermentation as shown in table 14 was carried out under identical conditions as described in example 3. The experimental results are tabulated in the Table 14 below:

Table 14

| Age of culture in the fermenters in hrs | Cell Concentration by measuring OD at 600 nm | | Yield of rhPDGF(% of total population) | |
|---|---|---|---|---|
| | Fermenter of Example 3b wherein production was induced and wherein glucose concentration in feed medium was 25% | Fermenter of Example 3 wherein production was induced and wherein glucose concentration in feed medium was 20% | Fermenter of Example 3b wherein production was induced and wherein glucose concentration in feed medium was 25% | Fermenter of Example 3 wherein production was induced and wherein glucose concentration in feed medium was 20% |
| 0 | 0.2 | 0.17 | NE[a] | NE |
| 16 | NS[b] | 52.6 | NS | 35.29 |
| 16.5 | 56.6 | NS | 39.36 | NS |
| 17 | NS | 51.2 | NS | 3 8.07 |
| 18 | 60.8 | 55.6 | 39 | 37.77 |
| 19 | 63 | 59 | 33.6 | 39.03 |
| 20 | 65.2 | 64 | 34.5 | 40.13 |
| 21 | 73.2 | 72.6 | 22.02 | 39.57 |
| 22 | 71 | 70 | 28.76 | 39.84 |
| NE[a] No expression NS[b] Not sampled | | | | |

It is seen from the table that reduction in the concentration of glucose (carbon source) in the feed medium led to an increased yield or expression of the desired protein rhPDGF by approximately 38%.

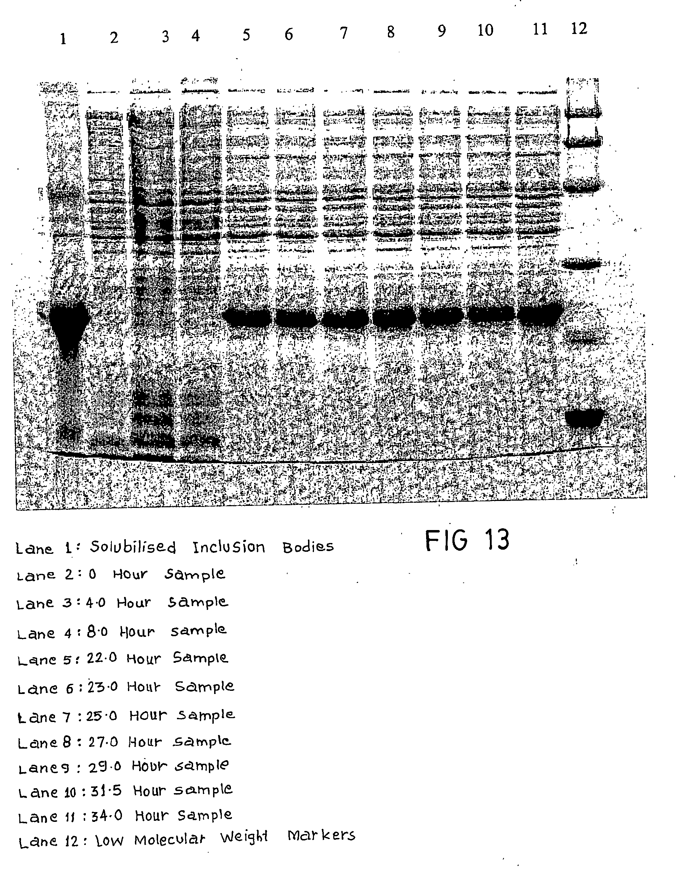

Fig 13 of the accompanying drawings shows SDS-PAGE analysis of culture samples of the fermentation of Example 3b for accumulation of rhPDGF. Lanes 1-9 correspond to the culture samples that were withdrawn from the fermentation broth of Example 3d at the time intervals in Table 5.

Example 3c

E Coli strain TOP10 transformed to express rhPDGF was purified and maintained in glycerol stocks. An aliquot of the culture was removed from the stock and streaked on 2XYT plate to separate single colonies after growth of 24 hours at 37°C. A single colony from the 2XYT plate was removed and inoculated into a falcon tube containing 10 ml of 2XYT liquid medium. After growth for 16 hours at 37°C on a rotary shaker (200 - 220 rpm), 5 ml of the culture from the tube was inoculated into a 500 ml conical flask containing 100 ml of the basal medium. After growth for 8 hours at 37°C on a rotary shaker (200 - 220 rpm), 100 ml of the culture from the flask was used to inoculate two jar fermenters (2 litres, B Braun) containing 900 ml of the basal medium. The fermentation conditions and duration of fermentation were the same as those in Example 3. The feed medium pumped into the fermenters and the feed strategy were the same as those in Example 3. Production in the fermenters was induced after 8 hours from the start of the fermentation by inoculation of the fermenters with inducer solutions containing concentrations of 1 % and 0.0 1 % of the inducer arabinose. Yields of 52% and 26% of rhPDGF were obtained in the fermenters induced with inducer of concentrations 1% and 0.01 %, respectively.

Bacterial density achieved and percentage of rhGH expressed in the two fermenters were compared. The experimental results were tabulated in the Table 15 below:

Table 15

| Age of culture in the fermenters in hrs | Cell concentration by measuring OD at 600 nm | | Yield of rhPDGF (% of total population) | |
|---|---|---|---|---|
| | Fermenter in which production was induced by inducer solution wherein concentra tion of the inducer was 1 % | Fermenter in which production was induced by inducer solution wherein concentra tion of the inducer was 0.01 % | Fermenter in which production was induced by inducer solution wherein concentra tion of the inducer was 1% | Fermenter in which production was induced by inducer solution wherein concentrat ion of the inducer was 0.01% |
| 0 | 0.16 | 0.21 | NE[a] | NE |
| 16 | 65.6 | 56.2 | 49.69 | 25.58 |
| 18 | NS[b] | 67 | NS | 26.59 |
| 19 | NS | 74.2 | NS | 19.17 |
| 20 | 86.2 | NS | 52.19 | NS |
| 24 | 97.8 | NS | 45.19 | NS |
| NE[a] No expression NS [b] Not sampled | | | | |

The experimental results show that irrespective of the concentration of the inducer in the inducer solution percentage of expression remained the same except that high levels of expressions in the two fermenters occurred at different points of time. Even though the concentration of the inducer was reduced by a factor of 100 the expression reduced only to half. This shows that various inducible promoters of varying strength and various optical density may be used in the fermentation process.

Example 4

E Coli strain TOP 10 transformed to express β-galactosidase was purified and maintained in glycerol stocks. An aliquot of the culture was removed from the stock and streaked on 2XYT plate to separate single colonies after growth of 24 hours at 37°C. A single colony from the 2XYT plate was removed and inoculated into a falcon tube containing 10 ml of 2XYT liquid medium. After growth for 16 hours at 37°C on a rotary shaker (200 - 220 rpm), 5 ml of the culture from the tube was inoculated into a 500 ml conical flask containing 100 ml of the basal medium. After growth for 8 hours at 37°C on a rotary shaker (200 - 220 rpm), 100 ml of the culture from the flask was used to innoculate ajar fermenter (2 litres, B Braun) containing 900 ml of the basal medium.

Fermentation was carried out in the fermenter at a temperature of 37°C and pH of the fermentation broth was maintained at pH7 using 12.5% of ammonia solution. The dissolved oxygen tension (dOT) was 50% air pressure for 16 hours of the fermentation time and was controlled by automatic adjustment of the fermenter stirrer speed. When $OD_{600}$ of approximately 1 was reached or at 2 hours after fermentation was started, the feed medium comprising glucose 25% (carbon source) and yeast extract 20% (nitrogen source) and further comprising FS4 solution (15 ml of FS4 solution for 600 ml of the feed medium) was pumped into the fermenter following a feed strategy as set out in Fig 1 of the accompanying drawings (high feed rate). The initial feed rate was 0.1 ml/hr and was raised exponentially till 18 mL/hr and thereafter linearly by a slope of 2.4. After 8 hours from start of the fermentation or when a cell concentration of $OD_{600}$ 15 was obtained, the fermenter was inoculated with an inducer solution containing 0.1 - 4% inducer namely arabinose between $OD_{600}$ of 15 to 40. Excessive foaming was controlled with the addition of antifoam solution (Dow Corning 1510, Antifoam). Fermentation was performed for 25 hours and during that time samples were taken for measurement of optical density and accumulation of β-galactosidase within the cells. β-galactosidase accumulation was measured by scanning Coomassie stained SDS-PAGE gels of whole cell lysates in known manner.

Yield of approximately 15-20% of β-galactosidase was obtained in the fermenter. The experimental results are tabulated in Table 16 below.

Table 16

| Growth of host E coli cells and expression of β-galactosidase during fermentation process of Example 4. | | |
|---|---|---|
| **Age of culture in the fermenter in hrs** | **Cell concentration by measuring OD at 600 nm in the fermenter wherein production was induced after 8 hours from the start of the fermentation** | **Yield of β-galactosidase (% of total population) in the fermenter wherein production was induced after 8 hours from the start of the fermentation** |
| 0 | 0.189 | NE[a] |
| 17 | 47 | 14.69 |
| 18 | 51.6 | 14.62 |
| 19 | 60 | 16.29 |
| 20 | 60.6 | 15.45 |
| 21 | 72.8 | 19.45 |
| 22 | 83.7 | 16.97 |
| 23 | 87 | 14.57 |
| 24 | 87.2 | 14.06 |
| NE[a] No expression | | |

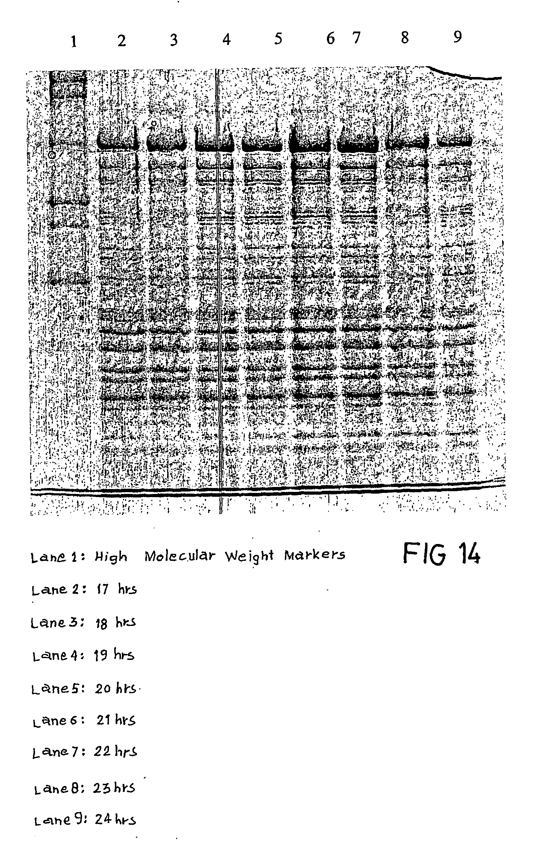

Fig 14 shows SDS-PAGE analysis of culture samples of the fermentation process of Example 4 for accumulation of β-galactosidase. Lanes 1-9 correspond to the culture samples that were withdrawn from the fermentation broth of Example 4 at the hours in Table 16.

The above examples show that heterologus recombinant proteins are produced by the process of the invention and the same feed medium is used for the entire fermentation process. The feed strategy employed is simple and easy to carry out and involves raising the initial feed rate exponentially and/or linearly using standard formulas. The acetate formed during the fermentation process of the invention is low and maintained at less than 1 gm/L. No equipments are used to control the formation of undesirable by-products thereby rendering the process economical and simple to carry out. Cells grow at a constant rate throughout the fermentation process. Also, the fermentation process of the invention ensures cell growth and expression of proteins simultaneously and independently throughout the entire process. Maximum cell concentration achieved is $OD_{600}$ of about 100. Due to reduced cell concentration, the biomass produced is also correspondingly reduced. Even though maximum cell concentration achieved is only $OD_{600}$ of about 100, the yield of the heterologous proteins is at least 3 gms/L. The above examples also show that various inducible promoters of varying strength may be used in the fermentation process of the invention.

**Claims**

1.    A low cell density fermentation process for production of heterologous proteins in microorganisms comprising :

a) feeding the cell culture obtained by cultivating host microorganisms transformed with a vector carrying genetic material for the said proteins and an inducible promoter under batch fermentation conditions, with a feed medium

after an $OD_{600}$ of 0.16 to 8 has been achieved or after 0 to 4 hrs from the start of the fermentation process, the feed medium comprising 5 to 30% of carbon source and 1 to 30% of nitrogen source and 0 mg to 400 mg antibiotics and 2.5 to 4.25% inorganic phosphates and trace elements, the concentration of the carbon source in the feed medium being 10 to 30 %, the amino acid content in nitrogen source being 45 to 95%, the initial feed rate being in the range of 0 ml/hr to 12 ml/hr and being raised exponentially by an exponent in the range of 0.1 to 0.4 and/or linearly with the slope of the curve in the range of 0.5 to 3; and

b) inducing production with 0.01 - 4% inducer at a cell density of $OD_{600}$ 0.1 - $OD_{600}$ 50,

feeding of the cell culture with the feed medium and feed rate of step (a) being continued after production has been induced and $pO_2$ being adjusted between 10% to 60% by passing sterile air into the fermentation broth and the temperature and pH of the fermentation broth being maintained at 33°C - 41°C and 6.9 - 8.5, respectively during the entire fermentation.

2. A process as claimed in claim 1, wherein the microorganism is E -Coli.

3. A process as claimed in claim 1, wherein the vector is a plasmid.

4. A process as claimed in claim 1, wherein the inducible vector is pET, pBAD or pTOPO or any other commercial inducible vector.

5. A process as claimed in claim 1, wherein the feeding of the cell culture with the feed medium is after an $OD_{600}$ of 0.15 to 4 has been achieved or after 1 to 2 hrs from the start of the fermentation process.

6. A process as claimed in claim 1, wherein the feed medium comprises 10 to 30% of carbon source and 10 to 30% of nitrogen source.

7. A process as claimed in claim 1, wherein the feed medium comprises 50mg to 400 mg of antibiotics and 3 to 4.25% of inorganic phosphates and trace elements.

8. A process as claimed in claim 1, wherein the antibiotic is ampicillin and inorganic phosphates and trace elements comprise salts of iron, zinc, cobalt, boron, copper or calcium.

9. A process as claimed in claim 1, wherein the carbon source is glucose or glycerol or mixture of glucose and glycerol.

10. A process as claimed in claim 1, wherein nitrogen source is yeast extract or soya peptone or tryptone or mixtures thereof.

11. A process as claimed in claim 1, wherein the amino acid content in the nitrogen source is in the range of 55% to 73% w/v.

12. A process as claimed in claim 1, wherein the initial feed rate is in the range of 3 to 12 ml/hr and is raised exponentially by an exponent in the range of 0.15 to 0.35 or linearly by a slope in the range of 0.75 to 2.8;

13. A process as claimed in claim 1, wherein the exponent is in the range of 0.2 to 0.3 or the slope is in the range of 1.2 to 2.4;

14. A process as claimed in claim 1, wherein the expression of heterologous proteins is induced at a cell density of $OD_{600}$ between 10 to 20.

15. A process as claimed in claim 1, wherein the production is induced with 0.01 to 2 % inducer.

16. A process as claimed in claim 1, wherein the inducer is arabinose.

17. A process as claimed in claim 1, wherein the pO2 is adjusted between 20% - 60%.

18. A process as claimed in claim 1, wherein the temperature of the fermentation broth is maintained at 37°C.

19. A process as claimed in claim 1, wherein the pH of the fermentation broth is maintained at 6.9 - 7.5.

EP 1 828 375 B1

**20.** A process as claimed in claim 1, wherein the pH of the fermentation broth is maintained at 6.95 - 8.

**21.** A process as claimed in claim 1, wherein the inducible promoter is T7 polymerase, uspA or araBAD or any other promoter present in commercial vectors.

**22.** A process as claimed in claim 1, wherein the heterologus proteins are recombinant Granulocyte Colony stimulating factors (rhG-CSFs), recombinant human growth hormone (rhGH), recombinant human Platelet Derived growth factor (rhPDGF) or β-galactosidase..

**Patentansprüche**

**1.** Fermentationsverfahren bei geringer Zelldichte zur Herstellung von heterologen Proteinen in Mikroorganismen, das umfasst:

a) Das Füttern der Zellkultur, welche durch Kultivieren von Wirtsorganismen erhalten wurde, die mit einem Vektor transformiert sind, der genetisches Material für die Proteine und einen unter Batch-Fermentationsbedingungen induzierbaren Promotor enthält, mit einem Feed-Medium, nachdem eine $OD_{600}$ von 0,16 bis 0,8 erreicht worden ist oder nach 0 bis 4 Stunden vom Beginn des Fermentationsprozesses, wobei das Feed-Medium 5 bis 30 % Kohlenstoffquelle und 1 bis 30 % Stickstoffquelle und 0 mg bis 400 mg Antibiotika und 2,5 bis 4,25 % anorganische Phosphate und Spurenelemente umfasst, die Konzentration von der Kohlenstoffquelle in dem Feed-Medium bei 10 bis 30 % liegt, der Aminosäuregehalt in der Stickstoffquelle 45 bis 95 % beträgt, die anfängliche Geschwindigkeit der Einspeisung in dem Bereich von 0 ml/Std. bis 12 ml/Std. liegt und exponentiell mit einem Exponenten im Bereich von 0,1 bis 0,4 und/oder linear mit einer Steigung der Kurve in einem Bereich von 0,5 bis 3 gesteigert wird; und

b) das Starten der Herstellung mit 0, 01 - 4 % Starter bei einer Zelldichte von $OD_{600}$ 0,1 - $OD_{600}$ 50, wobei das Füttern von der Zellkultur mit dem Zufütterungsmedium und der Geschwindigkeit der Einspeisung von Schritt (a) fortgeführt wird, nachdem die Herstellung gestartet worden ist und der $pO_2$ zwischen 10% und 60 % mittels Hindurchleiten von steriler Luft durch die Fermentationslösung eingestellt wurde und die Temperatur und der pH-Wert von der Fermentationslösung bei 33 °C - 41 °C, beziehungsweise bei pH 6,9 - 8,5 während der gesamten Fermentation aufrecht gehalten wird.

**2.** Verfahren nach Anspruch 1, wobei der Mikroorganismus E. coli ist.

**3.** Verfahren nach Anspruch 1, wobei der Vektor ein Plasmid ist.

**4.** Verfahren nach Anspruch 1, wobei der induzierbare Vektor pET, pBAD oder pTOPO oder irgend ein anderer handelsüblich erhältlicher, induzierbarer Vektor ist.

**5.** Verfahren nach Anspruch 1, wobei die Fütterung von der Zellkultur mit dem Zufütterungsmedium durchgeführt wird, nachdem eine $OD_{600}$ von 0,15 bis 4 erzielt worden ist oder nach 1 bis 2 Stunden nach dem Start von dem Fermentationsprozess.

**6.** Verfahren nach Anspruch 1, wobei das Zufütterungsmedium 10 bis 30 % der Kohlenstoffquelle und 10 bis 30 % der Stickstoffquelle umfasst.

**7.** Verfahren nach Anspruch 1, wobei das Zufütterungsmedium 50 mg bis 400 mg an Antibiotika und 3 bis 4,25 % an anorganischen Phosphaten und Spurenelementen umfasst.

**8.** Verfahren nach Anspruch 1, wobei das Antibiotikum Ampicillin ist und die anorganischen Phosphate und die Spurenelemente Salze von Eisen, Zink, Kobalt, Bor, Kupfer oder Calcium umfassen.

**9.** Verfahren nach Anspruch 1, wobei die Kohlenstoffquelle Glucose oder Glycerol oder eine Mischung aus Glucose und Glycerol ist.

**10.** Verfahren nach Anspruch 1, wobei die Stickstoffquelle ein Hefeextrakt oder Soja-Pepton oder Trypton oder Mischungen daraus ist.

24

**11.** Verfahren nach Anspruch 1, wobei der Gehalt an Aminosäuren in der Stickstoffquelle in dem Bereich von 55 Gew.-% bis 73 Gew.-% liegt.

**12.** Verfahren nach Anspruch 1, wobei die anfängliche Geschwindigkeit der Einspeisung in dem Bereich von 3 bis 12 ml/Std. liegt und exponentiell durch einen Exponenten gesteigert wird, der in dem Bereich von 0,15 bis 0,35 liegt oder linear mit einer Steigung der Geraden, die in dem Bereich von 0,75 bis 2 liegt.

**13.** Verfahren nach Anspruch 1, wobei der Exponent in dem Bereich von 0,2 bis 0,3 liegt oder die Steigung der Geraden sich in dem Bereich von 1,2 bis 2,4 befindet.

**14.** Verfahren nach Anspruch 1, wobei die Expression von den heterologen Proteinen bei einer Zelldichte von $OD_{600}$ zwischen 10 bis 20 induziert wird.

**15.** Verfahren nach Anspruch 1, wobei die Herstellung mit 0,01 bis 2 % Starter induziert ist.

**16.** Verfahren nach Anspruch 1, wobei der Starter Arabinose ist.

**17.** Verfahren nach Anspruch 1, wobei der $pO_2$ zwischen 20 % - 60 % eingestellt ist.

**18.** Verfahren nach Anspruch 1, wobei die Temperatur von der Fermentationslösung konstant bei 37 °C gehalten wird.

**19.** Verfahren nach Anspruch 1, wobei der pH-Wert von der Fermentationslösung zwischen pH 6,9 - 7,5 aufrecht gehalten wird.

**20.** Verfahren nach Anspruch 1, wobei der pH-Wert von der Fermentationslösung zwischen pH 6,95 - 8 aufrecht gehalten wird.

**21.** Verfahren nach Anspruch 1, wobei der induzierbare Promotor T7-Polymerase, uspA oder araBAD oder irgend ein anderer Promotor ist, wie er in handelsüblich erhältlichen Vektoren vorkommt.

**22.** Verfahren nach Anspruch 1, wobei die heterologen Proteine rekombinante Granulozyten-Kolonie stimulierende Faktoren (rhG-CSF), rekombinantes menschliches Wachstumshormon (rhGH), rekombinanter menschlicher Platelet Derived Growth Factor (rhPDGF) oder β-Galaktosidase sind.

**Revendications**

**1.** Procédé de fermentation à faible densité de cellules pour la production de protéines hétérologues dans des microorganismes, comprenant les étapes consistant à :

a) amener la culture de cellules obtenue par mise en culture de microorganismes hôtes transformés avec un matériau génétique portant un vecteur pour lesdites protéines et un promoteur inductible dans des conditions de fermentation de lots, avec un milieu d'amenée après l'obtention d'une valeur $OD_{600}$ de 0,16 à 8 ou après 0 à 4 h à partir du début du procédé de fermentation, le milieu d'amenée comprenant 5 à 30 % d'une source de carbone et 1 à 30 % d'une source d'azote et 0 mg à 400 mg d'antibiotique et 2,5 à 4,25 % de phosphates inorganiques et d'éléments à l'état de traces, la concentration de la source de carbone dans le milieu d'amenée étant de 10 à 30 %, la teneur en aminoacides dans la source d'azote étant de 45 à 95 %, la vitesse d'amenée initiale étant comprise dans la plage de 0 ml/h à 12 ml/h et augmentant de façon exponentielle d'un exposant dans la plage de 0,1 à 0,4 et/ou de façon linéaire avec la pente de la courbe dans la plage de 0,5 à 3 ; et
b) induire la production avec 0,01 à 4 % d'inducteur à une densité de cellules de $OD_{600}$ 0,1 à $OD_{600}$ 50,

amener la culture de cellules avec le milieu d'amenée et en continuant la vitesse d'amenée de l'étape (a) après que la production a été induite et en ajustant la valeur de $pO_2$ entre 10 % et 60 % en faisant passer de l'air stérile dans le bouillon de fermentation, et la température et le pH du bouillon de fermentation étant maintenus respectivement à 33 °C à 41 °C et à 6,9 à 8,5 pendant toute la fermentation.

**2.** Procédé selon la revendication 1, dans lequel le microorganisme est *E. coli*.

**3.** Procédé selon la revendication 1, dans lequel le vecteur est un plasmide.

**4.** Procédé selon la revendication 1, dans lequel le vecteur inductible est pET, pBAD ou pTOPO ou tout autre vecteur inductible du commerce.

**5.** Procédé selon la revendication 1, dans lequel on effectue l'amenée de la culture de cellules avec le milieu d'amenée après l'obtention d'une valeur de $OD_{600}$ de 0,15 à 4 ou après 1 à 2 h à partir du début du procédé de fermentation.

**6.** Procédé selon la revendication 1, dans lequel le milieu d'amenée comprend 10 à 30 % d'une source de carbone et 10 à 30 % d'une source d'azote.

**7.** Procédé selon la revendication 1, dans lequel le milieu d'amenée comprend 50 mg à 400 mg d'antibiotique et 3 à 4,25 % de phosphates inorganiques et d'éléments à l'état de traces.

**8.** Procédé selon la revendication 1, dans lequel l'antibiotique est l'ampicilline et les phosphates inorganiques et les éléments à l'état de traces comprennent des sels de fer, de zinc, de cobalt, de bore, de cuivre ou de calcium.

**9.** Procédé selon la revendication 1, dans lequel la source de carbone est du glucose ou du glycérol ou un mélange de glucose et de glycérol.

**10.** Procédé selon la revendication 1, dans lequel la source d'azote est un extrait de levure ou de la peptone de soja ou de la tryptone ou des mélanges de ceux-ci.

**11.** Procédé selon la revendication 1, dans lequel la teneur en aminoacides dans la source d'azote est comprise dans la plage de 55 % à 73 % p/v.

**12.** Procédé selon la revendication 1, dans lequel la vitesse d'amenée initiale est comprise dans la plage de 3 à 12 ml/h et augmente de façon exponentielle d'un exposant compris dans la plage de 0,15 à 0,35 ou de façon linéaire d'une pente comprise dans la plage de 0,75 à 2,8.

**13.** Procédé selon la revendication 1, dans lequel l'exposant est compris dans la plage de 0,2 à 0,3 ou la pente est comprise dans la plage de 1,2 à 2,4.

**14.** Procédé selon la revendication 1, dans lequel l'expression de protéines hétérologues est induite à une densité de cellules de $OD_{600}$ comprise entre 10 et 20.

**15.** Procédé selon la revendication 1, dans lequel la production est induite avec 0,01 à 2 % d'inducteur.

**16.** Procédé selon la revendication 1, dans lequel l'inducteur est de l'arabinose.

**17.** Procédé selon la revendication 1, dans lequel on ajuste la valeur de $pO_2$ entre 20 % et 60 %.

**18.** Procédé selon la revendication 1, dans lequel on maintient la température du bouillon de fermentation à 37 °C.

**19.** Procédé selon la revendication 1, dans lequel on maintient le pH du bouillon de fermentation à 6,9 à 7,5.

**20.** Procédé selon la revendication 1, dans lequel on maintient le pH du bouillon de fermentation à 6,95 à 8.

**21.** Procédé selon la revendication 1, dans lequel le promoteur inductible est la T7 polymérase, uspA ou araBAD ou tout autre promoteur présent dans des vecteurs du commerce.

**22.** Procédé selon la revendication 1, dans lequel les protéines hétérologues sont les facteurs recombinants stimulant la formation de colonies de granulocytes (rhG-CSF), l'hormone de croissance humaine recombinante (rhGH), le facteur de croissance recombinant dérivé de plaquettes humaines (rhPDGF) ou la β-galactosidase.

FIG 1

FIG 2

FIG 3

Lane 1 : Molecular Weight marker  FIG 4

Lane 2 : 0-hr after production is induced

Lane 3 : 16-hr

Lane 4 : 17-hr

Lane 5 : 18-hr

Lane 6 : 19-hr

Lane 7 : 20-hr

Lane 8 : 21-hr

Lane 9 : 22-hr

Lane 10 : 23-hr

FIG 5

FIG 6

Lane 1 : Molecular weight marker
Lane 2 : 0-hr after production is induced
Lane 3 : 16-hr.
Lane 4 : 17-hr
Lane 5 : 18-hr
Lane 6 : 19-hr
Lan 7 : 20-hr
Lane 8 : 21-hr
Lane 9 : 22-hr
Lane 10 : 23-hr
Lane 11 : 24-hr

FIG 7

FIG 8

FIG 9

FIG 10

FIG 11

FIG 12

FIG 13

Lane 1: Solubilised Inclusion Bodies

Lane 2: 0 Hour Sample

Lane 3: 4.0 Hour Sample

Lane 4: 8.0 Hour sample

Lane 5: 22.0 Hour Sample

Lane 6: 23.0 Hour Sample

Lane 7: 25.0 Hour Sample

Lane 8: 27.0 Hour Sample

Lane 9: 29.0 Hour sample

Lane 10: 31.5 Hour sample

Lane 11: 34.0 Hour Sample

Lane 12: Low Molecular Weight Markers

Lane 1: High Molecular Weight Markers

Lane 2: 17 hrs

Lane 3: 18 hrs

Lane 4: 19 hrs

Lane 5: 20 hrs

Lane 6: 21 hrs

Lane 7: 22 hrs

Lane 8: 23 hrs

Lane 9: 24 hrs

FIG 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0142420 A
- US 5714348 A
- US 5595905 A
- WO 0240697 A

- WO 9639523 A
- WO 02077205 A2
- EP 0755438 A

**Non-patent literature cited in the description**

- **SCHWEDER T et al.** Role of the general stress response during strong overexpression of a heterologous gene in Escherichia coli. *Appl. Microbiol Biotech,* 2002, vol. 58, 330-337
- **LIN HY ; NEUBAUER P.** *J Biotechnology,* 2000, vol. 79 (1), 27-37
- **KLEMAN G L et al.** *Appl. Environ. Microbiol,* 1991, vol. 57 (4), 910-917
- **CHANG C C et al.** *Appl. Microbiol Biotechnol,* 1998, vol. 49 (5), 531-537
- **CHEN Q et al.** *Appl. Biochem. Biotechnol,* 1995, vol. 51 - 52, 449-461

- **LEE, J.Y. ; YOON, C.S. et al.** Scale-up process for expression and renaturation of recombinant human epidermal growth factor from Escherichia coli inclusion bodies. *Biotechnol Appl Biochem,* 2000, vol. 31, 245-248
- **SHIMIZU, N. ; FUKUZONO, S. et al.** *Biotechnol. Bioeng.,* 1991, vol. 38, 37-42
- **TSAI, L.B. ; MANN, M. et al.** *J. Ind. Microbiol.,* 1987, vol. 2, 181-187
- **LEE S.Y.** *Trends. Biotech.,* 1996, vol. 14, 98-105